(19) Europäisches Patentamt
European Patent Office
Office européen des brevets

(11) EP 0 998 452 B1

(12) EUROPÄISCHE PATENTSCHRIFT

(45) Veröffentlichungstag und Bekanntmachung des Hinweises auf die Patenterteilung:
**18.05.2005 Patentblatt 2005/20**

(21) Anmeldenummer: **98942550.9**

(22) Anmeldetag: **14.07.1998**

(51) Int Cl.[7]: **C07C 255/34**, C09K 19/30, C09K 19/12, C09K 19/20, C09K 19/34, C07C 255/35, C07D 213/24, C07D 239/26, C07D 319/06

(86) Internationale Anmeldenummer:
**PCT/EP1998/004366**

(87) Internationale Veröffentlichungsnummer:
**WO 1999/005097 (04.02.1999 Gazette 1999/05)**

(54) **FLÜSSIGKRISTALLINE PROPEN- ODER PROPYNYLNITRILDERIVATE**
LIQUID CRYSTAL PROPENE OR PROPENYL NITRILE DERIVATIVES
DERIVES DE NITRILE DE PROPENE OU DE PROPENYLE A CRISTAUX LIQUIDES

(84) Benannte Vertragsstaaten:
**CH DE GB LI NL**

(30) Priorität: **24.07.1997 DE 19731906**
**29.09.1997 DE 19742898**

(43) Veröffentlichungstag der Anmeldung:
**10.05.2000 Patentblatt 2000/19**

(73) Patentinhaber: **MERCK PATENT GmbH**
**64293 Darmstadt (DE)**

(72) Erfinder:
- **REIFFENRATH, Volker**
  **D-64380 Rossdorf (DE)**
- **HIRSCHMANN, Harald**
  **D-64291 Darmstadt (DE)**

(56) Entgegenhaltungen:
**EP-A- 0 310 676** **EP-A- 0 816 332**
**GB-A- 2 111 992**

- **CHEMICAL ABSTRACTS, vol. 102, no. 25, 24. Juni 1985 Columbus, Ohio, US; abstract no. 220593, "Phenylpropiolnitrile derivatives" XP002040645 & JP 60 019756 A (CHISSO CORP.;JAPAN) 31. Januar 1985**
- **CHEMICAL ABSTRACTS, vol. 104, no. 18, 5. Mai 1986 Columbus, Ohio, US; abstract no. 160058, IIDA H ET AL: "Propynenitrile derivatives" XP002040646 & JP 60 169455 A (MITSUBISHI CHEMICAL INDUSTRIES CO., LTD.;JAPAN) 2. September 1985**
- **CHEMICAL ABSTRACTS, vol. 104, no. 14, 7. April 1986 Columbus, Ohio, US; abstract no. 120516, IIDA H ET AL: "Propynenitrile derivatives for liquid-crystal compositions" XP002040647 & JP 60 188358 A (MITSUBISHI CHEMICAL INDUSTRIES CO., LTD.;JAPAN) 25. September 1985**
- **CHEMICAL ABSTRACTS, vol. 102, no. 1, 7. Januar 1985 Columbus, Ohio, US; abstract no. 005964, "4-Acyloxyphenylpropiolonitriles" XP002040648 & JP 59 139353 A (CHISSO CORP.;JAPAN) 10. August 1984**
- **CHEMICAL ABSTRACTS, vol. 097, no. 9, 30. August 1982 Columbus, Ohio, US; abstract no. 072113, "Cinnamonitriles" XP002084194 & JP 57 070851 A (ASAHI GLASS CO., LTD.;JAPAN) 1. Mai 1982**
- **CHEMICAL ABSTRACTS, vol. 093, no. 13, 29. September 1980 Columbus, Ohio, US; abstract no. 132157, KITAMURA T ET AL: "Cyclohexylcinnamonitriles" XP002084195 in der Anmeldung erwähnt & JP 55 009012 - (HITACHI, LTD.;JAPAN) 22. Januar 1980**
- **CHEMICAL ABSTRACTS, vol. 102, no. 19, 13. Mai 1985 Columbus, Ohio, US; abstract no. 166483, "4-Pentylbiphenylyl-4'-propionitrile" XP002084196 & JP 59 190958 A (CHISSO CORP.;JAPAN) 29. Oktober 1984**
- **CHEMICAL ABSTRACTS, vol. 097, no. 11, 13. September 1982 Columbus, Ohio, US; abstract no. 091973, "Cinnamonitriles" XP002084197 & JP 57 035552 A (ASAHI GLASS CO., LTD.;JAPAN) 26. Februar 1982**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 998 452 B1

- **CHEMICAL ABSTRACTS, vol. 093, no. 9, 1. September 1980 Columbus, Ohio, US; abstract no. 095021, KITAMURA T ET AL: "n-Alkyl(and alkyloxy)phenyl-p'-trans-cinnamonitriles" XP002084198 in der Anmeldung erwähnt & JP 55 011529 - (HITACHI, LTD.;JAPAN) 26. Januar 1980**


Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft Acetylenderivate der Formel I

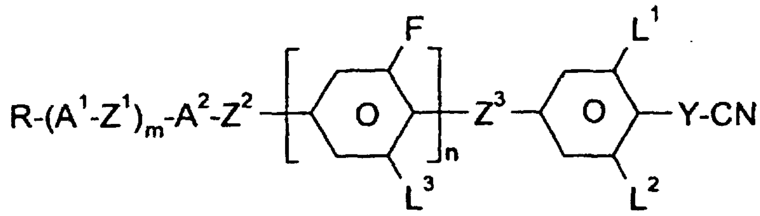

I

worin

R einen unsubstituierten, einen einfach durch CN oder $CF_3$ oder einen mindestens einfach durch Halogen substituierten Alkyl- oder Alkenylrest mit 1 bis 15 C-Atomen, wobei in diesen Resten auch eine oder mehrere $CH_2$-Gruppen jeweils unabhängig voneinander durch -O-, -S-,

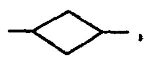

-CO-, -CO-O-, -O-CO- oder -O-CO-O- so ersetzt sein können, daß O-Atome nicht direkt miteinander verknüpft sind,

$A^1$ und $A^2$ jeweils unabhängig voneinander

(a) trans-1,4-Cyclohexylenrest, worin auch eine oder mehrere nicht benachbarte $CH_2$-Gruppen durch -O- und/oder -S- ersetzt sein können,

(b) 1,4-Phenylenrest, worin auch eine oder zwei CH-Gruppen durch N ersetzt sein können,

(c) trans-1,4-Cyclohexenylen,

(d) Rest aus der Gruppe 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,

wobei die Reste (a) bis (c) durch Fluor oder $CH_3$ ein- oder zweifach substituiert sein können,

$Z^1$, $Z^2$ und $Z^3$ jeweils unabhängig voneinander -CO-O-, -O-CO-, $-CH_2O-$, $-OCH_2-$, $-CH_2CH_2-$, -CH=CH-, -C≡C-, $-(CH_2)_4-$, $-OCF_2-$, $-CF_2O-$, $-CH=CH-CH_2CH_2-$ oder eine Einfachbindung,

m 0, 1 oder 2,

n 0 oder 1,

$L^1$, $L^2$ und $L^3$ jeweils unabhängig voneinander H oder F,

Y -C≡C-, -CH=CH-, -CF=CH-, -CH=CF- oder -CF=CF-

bedeuten, mit der Maßgabe, daß im Fall Y = -C≡C- oder -CH=CH-

a) n = 1 und $Z^3$ eine Einfachbindung, oder

b) $L^1$ und/oder $L^2$ Fluor, oder

c) R Alkenyl oder Alkenyloxy mit 2 bis 12 C-Atomen, oder

d) $A^2$ einen Pyrimidinrest oder einen mit Fluor oder $CH_3$ ein- oder zweifach substituierten trans-1,4-Cyclohexylenrest, oder

e) einer der Reste $Z^1$, $Z^2$ oder $Z^3$ -CO-O-
bedeutet, und im Fall Y = -C≡C- zusätzlich

f) n = 1 und $L^3$ = F oder

g) mindestens einer der Reste $Z^1$, $Z^2$ und $Z^3$ $-CF_2O-$ oder $-OCF_2-$ oder

h) $A^2$ 3,5-Difluor-1 ,4-phenylen bedeutet.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine relativ hohe dielektrische Anisotropie.

Es wurde nun gefunden, daß Verbindungen der Formel I als Komponenten flüssigkristalliner Medien vorzüglich geeignet sind. Insbesondere verfügen sie über hohe $\Delta\varepsilon$-Werte bei gleichzeitig hohen Klärpunkten. Mit ihrer Hilfe lassen sich stabile flüssigkristalline Medien mit breitem Mesophasenbereich und vorteilhaften Werten für die optische und dielektrische Anisotropie erhalten. Diese Medien weisen femer ein sehr gutes Tieftemperaturverhalten auf.

Verbindungen der Formel

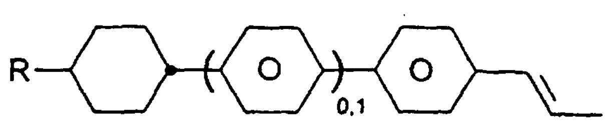

R = Alkyl

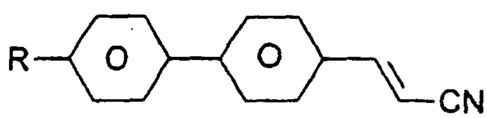

sind beispielsweise aus der JP 55-149371, JP 55-009012, JP 55-011529, PL 137 995 und PL 138 286 bekannt.

Cyanoethinderivate der Formel

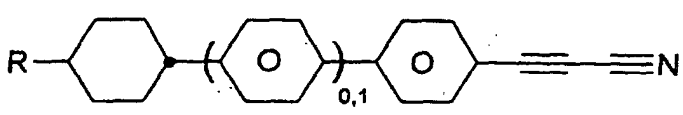

R = H oder Alkyl oder Alkyl
sind Gegenstand der JP 59-190 958 A2, JP 60-169 455 A2, JP 60-188 358 A2 und JP 60-019 756 A2.

Biphenylacetylene der Formel

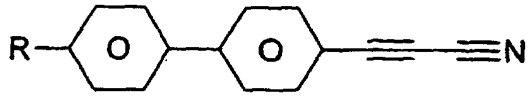

R = Alkyl werden in der DE 32 46 440 A1 beschrieben.

Die erfindungsgemäßen fluorierten Verbindungen werden dort aber nicht genannt.

Weitere Propenyl- und Propynylnitrilderivate sind aus EP 0 816 332 A1, GB 2 111 992 A, EP 0 310 676 A1, JP 59-139353 A (CA 5964b), JP 57-070851 A (CA 72113q), JP 57-035552 A (CA 91973m), JP 60-169455 A (CA 160058u) und JP 55-009012 A (CA 132157m) bekannt. Erfindungsgemäße Verbindungen werden darin jedoch nicht beschrieben.

Im Hinblick auf die verschiedensten Einsatzbereiche derartiger Verbindungen mit hohem $\Delta\varepsilon$ war es jedoch wünschenswert, weitere Verbindungen mit hoher Nematogenität zur Verfügung zu haben, die auf die jeweiligen Anwendungen genau maßgeschneiderte Eigenschaften aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden in der Regel flüssigkristalline Mesophasen in einem für die elektrooptische Verwendung günstig gelegenen Temperaturbereich. Chemisch und thermisch sind die erfindungsgemäßen Verbindungen stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

Der Einfachheit halber bedeuten im folgenden A4 bzw. A3 einen Rest der Formel

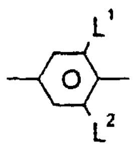

bzw.

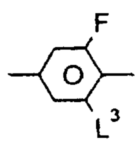

,

Cyc einen 1,4-Cyclohexylenrest, Che einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bi einen Bicyclo(2,2,2)-octylenrest, wobei Cyc und/oder Phe unsubstituiert oder ein- oder zweifach durch F oder CN substituiert sein können.

$A^1$ und $A^2$ sind vorzugsweise ausgewählt aus der Gruppe Cyc, Che, Phe, Pyr, Pyd und Dio.

Die Verbindungen der Formel I umfassen dementsprechend Verbindungen mit zwei Ringen der Teilformeln Ia und Ib:

$$R\text{-}A^2\text{-}A^4\text{-}Y\text{-}CN \qquad \text{Ia}$$

$$R\text{-}A^2\text{-}Z^2\text{-}A^4\text{-}Y\text{-}CN \qquad \text{Ib}$$

Verbindungen mit drei Ringen der Teilformeln Ic is Ig

$$R\text{-}A^1\text{-}A^2\text{-}A^4\text{-}Y\text{-}CN \qquad \text{Ic}$$

$$R\text{-}A^1\text{-}Z^1\text{-}A^2\text{-}A^4\text{-}Y\text{-}CN \qquad Id$$

$$R\text{-}A^1\text{-}A^2\text{-}Z^2\text{-}A^4\text{-}Y\text{-}CN \qquad Ie$$

$$R\text{-}A^1\text{-}A^3\text{-}A^4\text{-}Y\text{-}CN \qquad If$$

$$R\text{-}A^1\text{-}Z^1\text{-}A^3\text{-}A^4\text{-}Y\text{-}CN \qquad Ig$$

sowie Verbindungen mit vier Ringen der Teilformeln Ih bis Ik:

$$R\text{-}A^1\text{-}A^2\text{-}A^3\text{-}A^4\text{-}Y\text{-}CN \qquad Ih$$

$$R\text{-}A^1\text{-}Z^1\text{-}A^2\text{-}A^3\text{-}A^4\text{-}Y\text{-}CN \qquad Ii$$

$$R\text{-}A^1\text{-}A^2\text{-}Z^2\text{-}A^3\text{-}A^4\text{-}Y\text{-}CN \qquad Ij$$

$$R\text{-}A^1\text{-}Z^1\text{-}A^2\text{-}Z^2\text{-}A^3\text{-}A^4\text{-}Y\text{-}CN \qquad Ik$$

Y bedeutet vorzugsweise -C≡C-, ferner -CF=CF- oder -CF=CH-.

Bevorzugt sind auch Verbindungen der Formel I sowie aller Teilformeln, in denen $A^1$, $A^2$, $A^3$ und/oder $A^4$ ein- oder zweifach durch F substituiertes 1,4-Phenylen bedeutet. Insbesondere sind dies 2-Fluor-1,4-phenylen, 3-Fluor-1,4-phenylen und 3,5-Difluor-1,4-phenylen.

$A^1$ und $A^2$ sind vorzugsweise

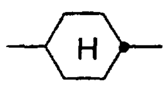 , 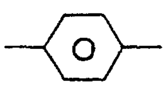 , 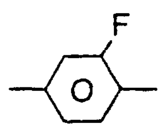 ,

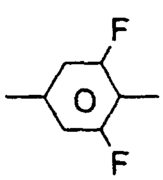 , 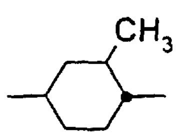 , 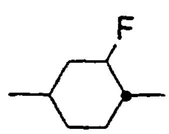 , 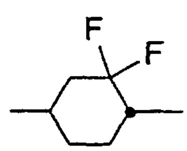 , 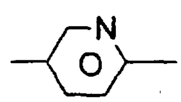 , 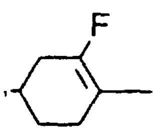 ,

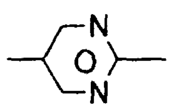 oder 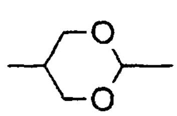 .

$Z^1$, $Z^2$ und $Z^3$ bedeuten bevorzugt eine Einfachbindung, -CO-O-, -O-CO- und $-CH_2CH_2-$, in zweiter Linie bevorzugt $-CF_2O-$, $-OCF_2-$, $-CH_2O-$ und $-OCH_2-$. Falls einer der Reste $Z^1$, $Z^2$ und $Z^3$-$(CH_2)_4$- oder $-CH=CH-CH_2CH_2-$ bedeutet, so ist der andere Rest $Z^1$ oder $Z^2$ bzw. $Z^3$ (falls vorhanden) vorzugsweise eine Einfachbindung.

m und n sind vorzugsweise 1 oder 0, insbesondere bevorzugt sind Verbindungen mit m = n = 0, ferner mit m

+ n = 1.

Falls R einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine $CH_2$-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine $CH_2$-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonyl-methyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkylrest bedeutet, in dem eine $CH_2$-Gruppe durch unsubstituiertes oder substituiertes -CH=CH- und eine benachbarte $CH_2$-Gruppe durch CO oder CO-O oder O-CO ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R einen einfach durch CN oder $CF_3$ substituierten Alkyl- oder Alkenylrest bedeutet, so ist dieser Rest vorzugsweise geradkettig und die Substitution durch CN oder $CF_3$ in $\bar{\omega}$-Position.

Falls R einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I, die über für Polymerisationsreaktionen geeignete Flügelgruppen R verfügen, eignen sich zur Darstellung flüssigkristalliner Polymerer.

Verbindungen der Formel I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit $S_A$-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2 Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R einen Alkylrest darstellt, in dem zwei oder mehr $CH_2$-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxyethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxy-carbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl,

Verbindungen der Formel I, die über für Polykondensationen geeignete Flügelgruppen R verfügen, eignen sich zur Darstellung flüssigkristalliner Polykondensate.

Formel I umfaßt sowohl die Racemate dieser Verbindungen als auch die optischen Antipoden sowie deren

Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Bevorzugte kleinere Gruppen von Verbindungen sind diejenigen der Teilformeln I1 bis I37:

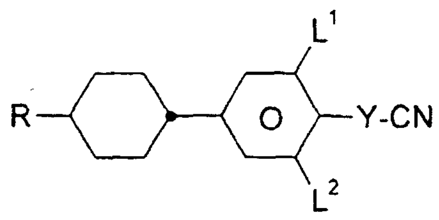

I1

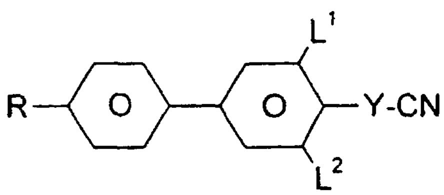

I2

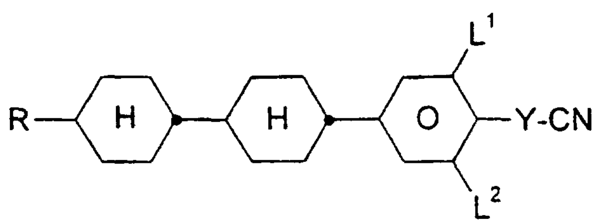

I3

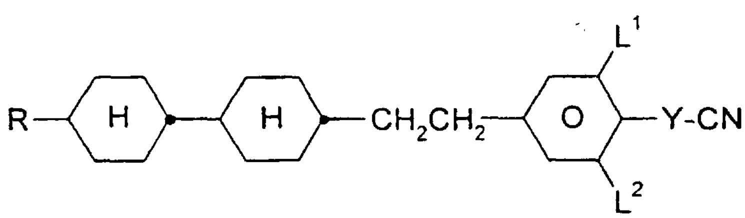

I4

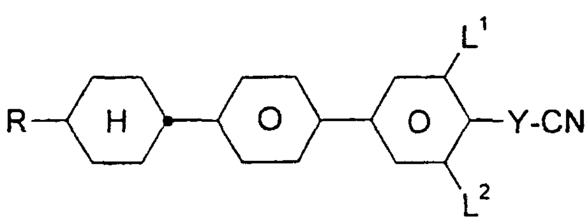

I5

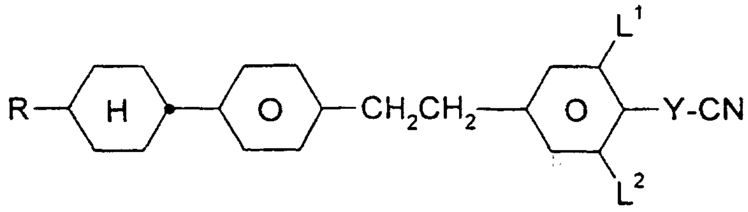

I6

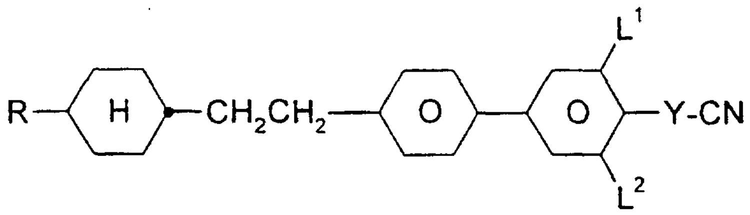

I7

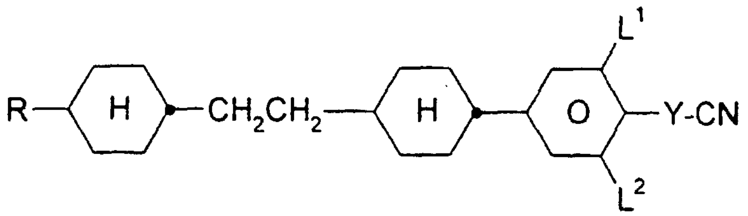

I8

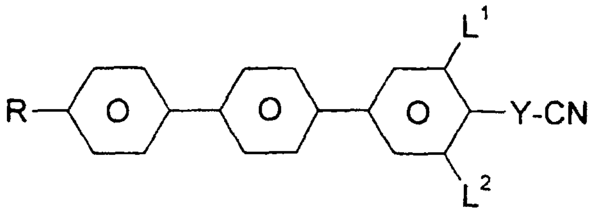

I9

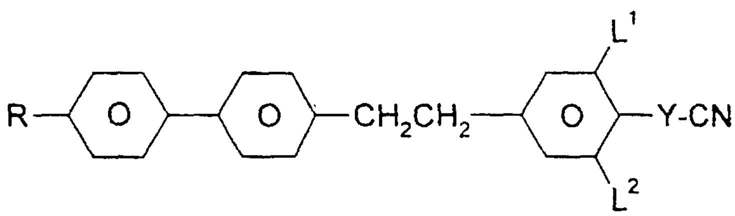

I10

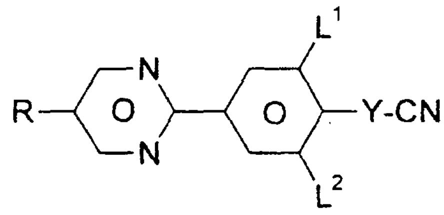

I11

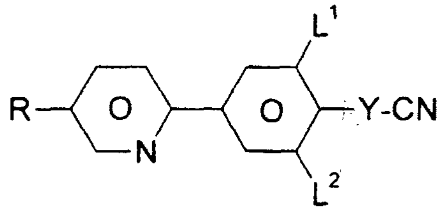

I12

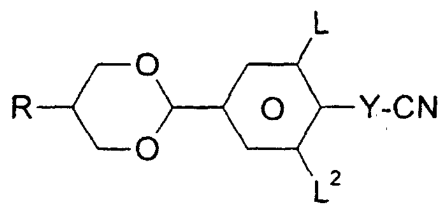

I13

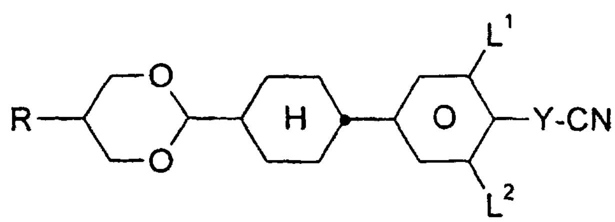

I14

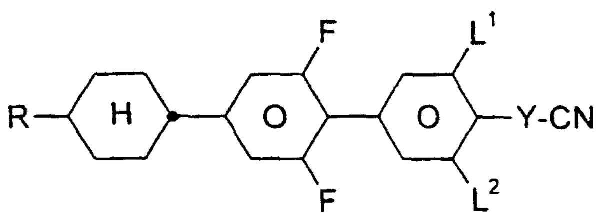

I15

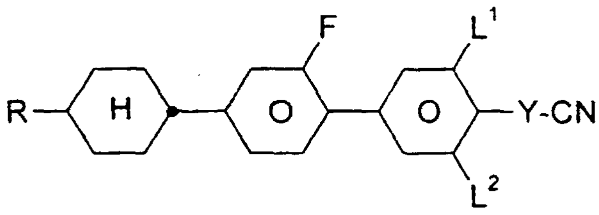

I16

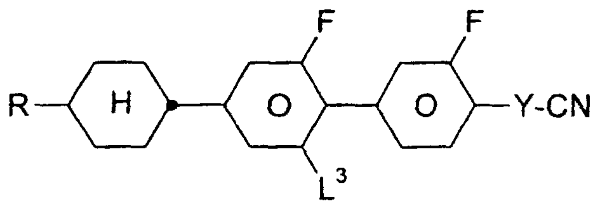

I17

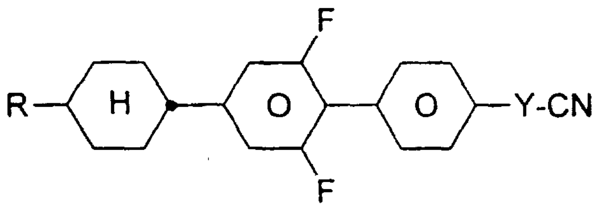

I18

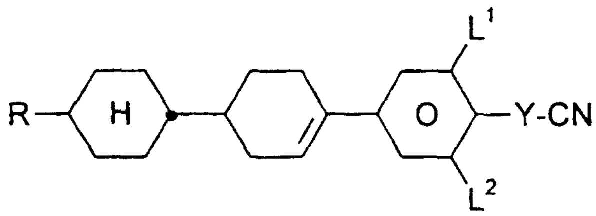

I19

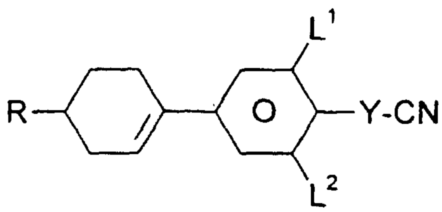

I20

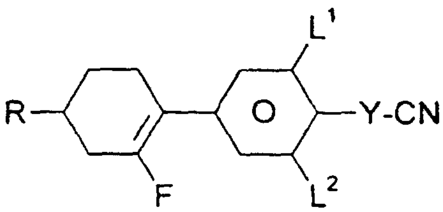

I21

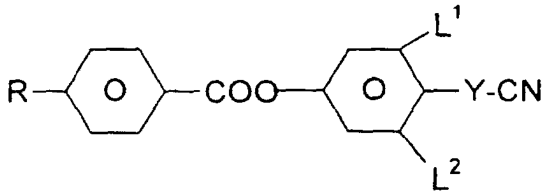

I22

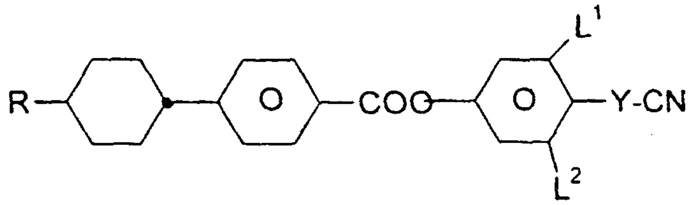

I23

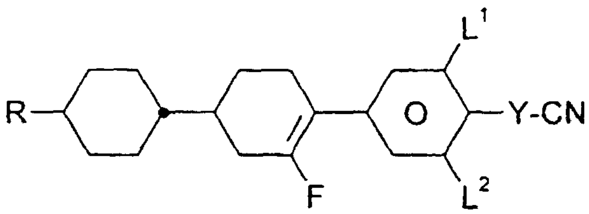

I24

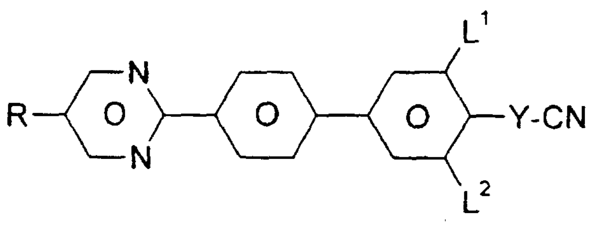

I25

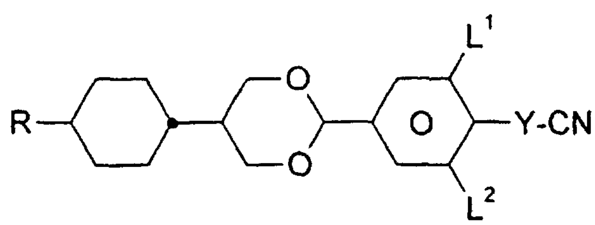

I26

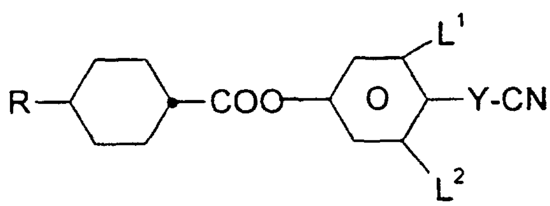

I27

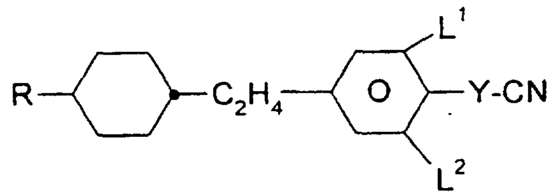

I28

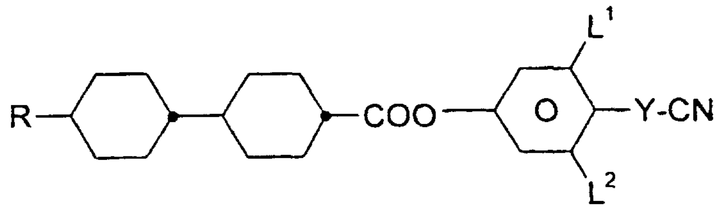

I29

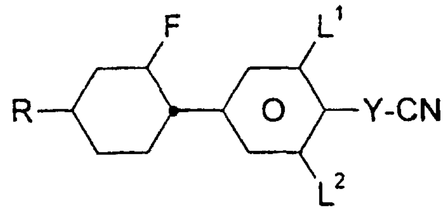

I30

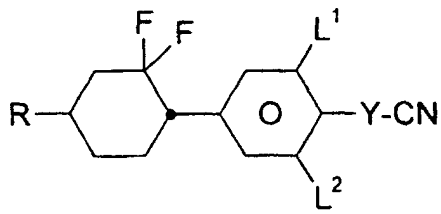

I31

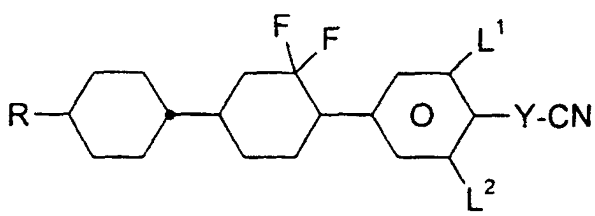

I32

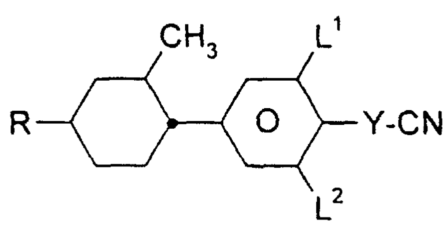

I33

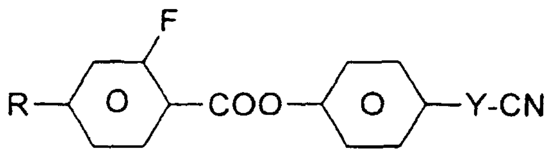

I34

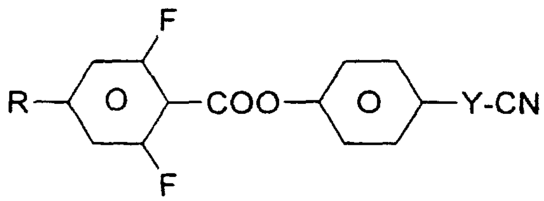

I35

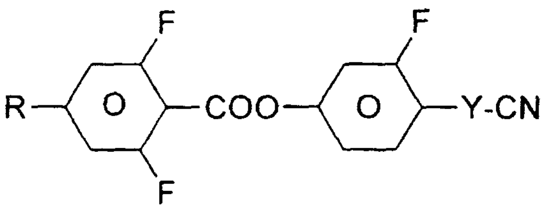

I36

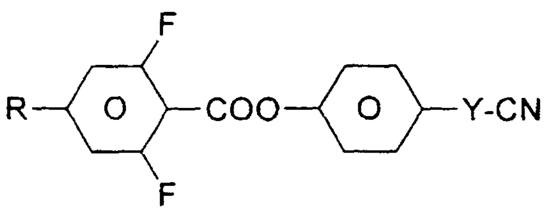

I37

Besonders bevorzugte Verbindungen sind Verbindungen der Unterformeln I1, I2, I3, I4, I5, I8, I11, I13, I16, I21, I30, I31, I33, I34, I35, I36 und I37.

In den Unterformeln 11-137 bedeutet Y vorzugsweise -C≡C- und -CF=CF-, ferner -CF=CH-. R ist vorzugsweise geradkettiges Alkyl, Alkoxy, 1 E- bzw. 3E-Alkenyl mit bis zu 6 C-Atomen, ferner 2Z-Alkenyl, 4-Alkenyl oder Alkenyloxy.

Im Rahmen der vorliegenden Erfindung umfaßt der Ausdruck "1E-Alkenyl" Reste wie Vinyl, 1E-Propenyl, 1E-Butenyl, 1E-Pentenyl, 1E-Hexenyl, 1E-Heptenyl, 1E-Octenyl, 1E-Nonenyl und 1E-Decenyl. Der Ausdruck "2Z-Alkenyl" umfaßt Reste wie Allyl, 2Z-Butenyl, 2Z-Pentenyl, 2Z-Hexenyl, 2Z-Heptenyl, 2Z-Octenyl, 2Z-Nonenyl und 2Z-Decenyl. Der Ausdruck "3E-Alkenyl" umfaßt Reste wie 3-Butenyl, 3E-Pentenyl, 3E-Hexenyl, 3E-Heptenyl, 3E-Octenyl, 3E-Nonenyl und 3E-Decenyl. Der Ausdruck "4-Alkenyl" umfaßt Reste wie 4-Pentenyl und die E- und/oder Z-Form von 3-Hexenyl, 4-Heptenyl, 4-Octenyl, 4-Nonenyl und 4-Decenyl.

Der Ausdruck "Alkenyloxy" bezeichnet Alkenyloxygruppen, in denen der Sauerstoff mit einem gesättigten Kohlenstoffatom direkt verknüpft ist (d.h. Gruppen welche ein oder mehrere Kohlenstoffatome zwischen der Doppelbindung und dem Sauerstoffatom aufweisen), wie (2E-Alkenyl)oxy, (3-Alkenyl)oxy, (4-Alkenyl)oxy, (5-Alkenyl)oxy und dergleichen. Der Ausdruck "(2E-Alkenyloxy)" umfaßt hierbei Reste wie Allyloxy, (2E-Butenyl)oxy, (2E-Pentenyl)oxy, (2E-Hexenyl)oxy, (2E-Heptenyl)oxy, (2E-Octenyl)oxy, (2E-Nonenyl)oxy und (2E-Decenyl)oxy. Der Ausdruck "(3-Alkenyl)oxy" umfaßt Reste wie (3-Butenyl)oxy und die E- und/oder Z-Form von (3-Pentenyl)oxy, (3-Hexenyl)oxy, (3-Heptenyl)oxy, (3-Octenyl)oxy, (3-Nonenyl)oxy und (3-Decenyl)oxy. Der Ausdruck "(4-Alkenyl)oxy" umfaßt Reste wie (4-Pentenyl)oxy und die E- und/oder Z-Form von (4-Hexenyl)oxy, (4-Heptenyl)oxy, (4-Octenyl)oxy, (4-Nonenyl)oxy und (4-Decenyl)oxy. Der Ausdruck "(5-Alkenyl)oxy" umfaßt Reste wie (5-Hexenyl)oxy und die E- und/oder Z-Form von (5-Heptenyl)oxy, (5-Octenyl)oxy, (5-Nonenyl)oxy und (5-Decenyl)oxy.

Es versteht sich für den Fachmann von selbst, daß Anspruch 1 ebenfalls alle Verbindungen der Formel I umfaßt, worin die Atome H, N, O, C, F durch ihre Isotope ersetzt sind.

Die 1,4-Cyctohexenylen-Gruppe hat vorzugsweise folgende Strukturen:

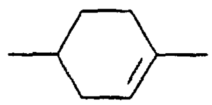 , 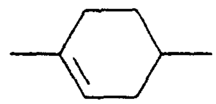

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind.

Dabei kann man auch von an sich bekannten, hier nicht näher erwähnten Varianten Gebrauch machen.

Schema 1

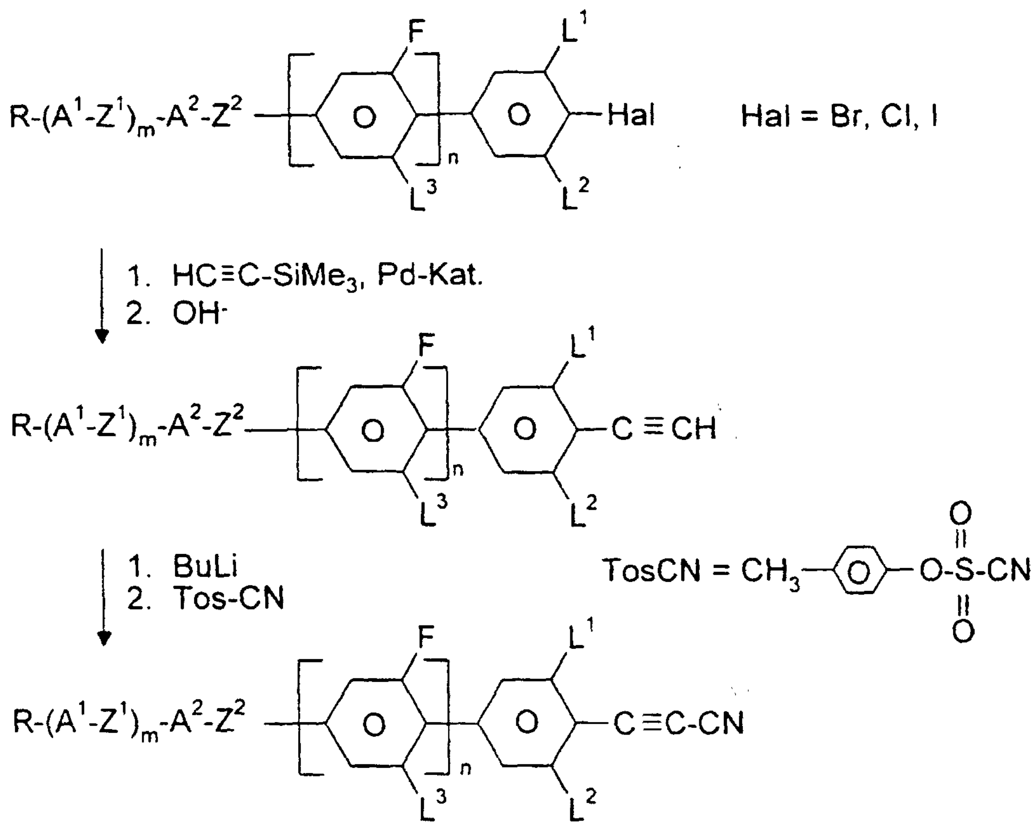

Schema 2

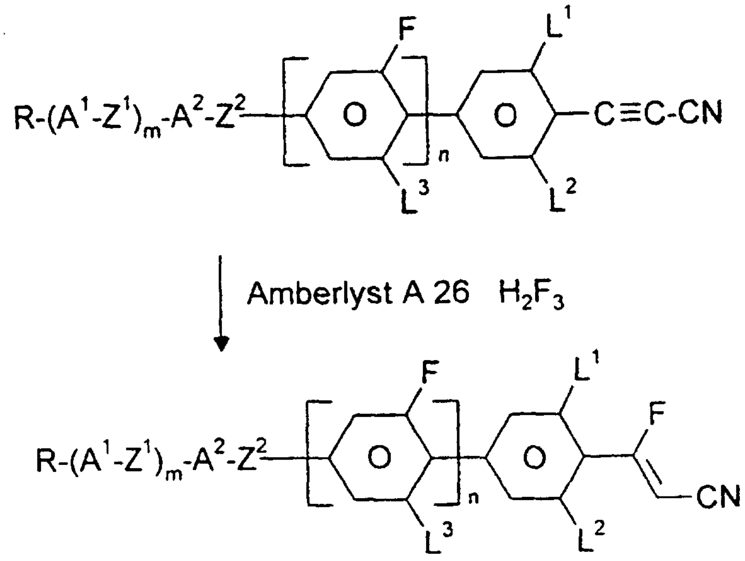


Schema 3

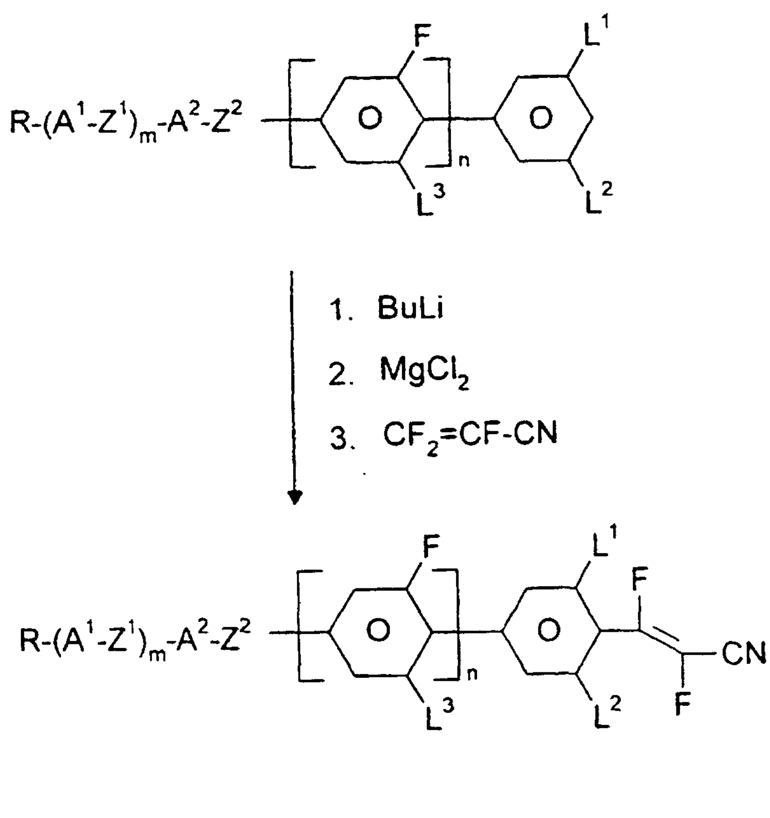

Schema 4

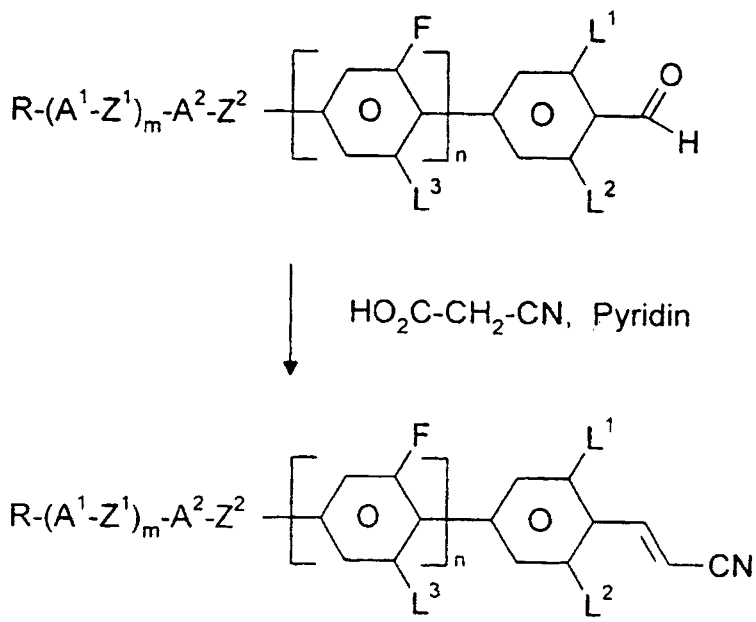


Bevorzugte Verbindungen werden beispielsweise wie folgt hergestellt:

Schema 5

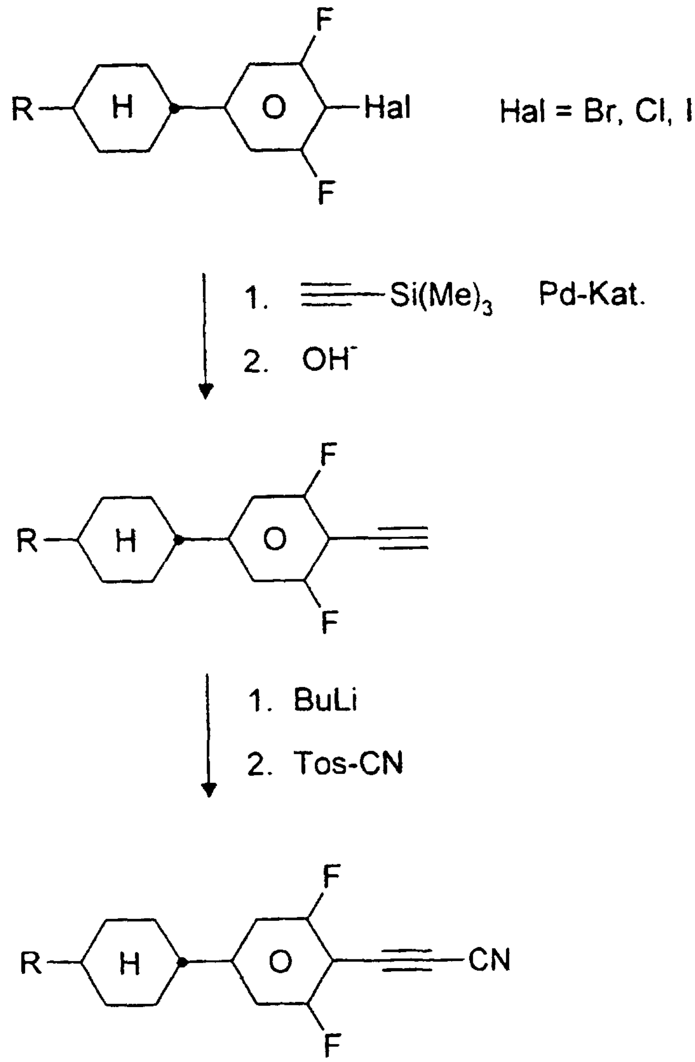

Schema 6

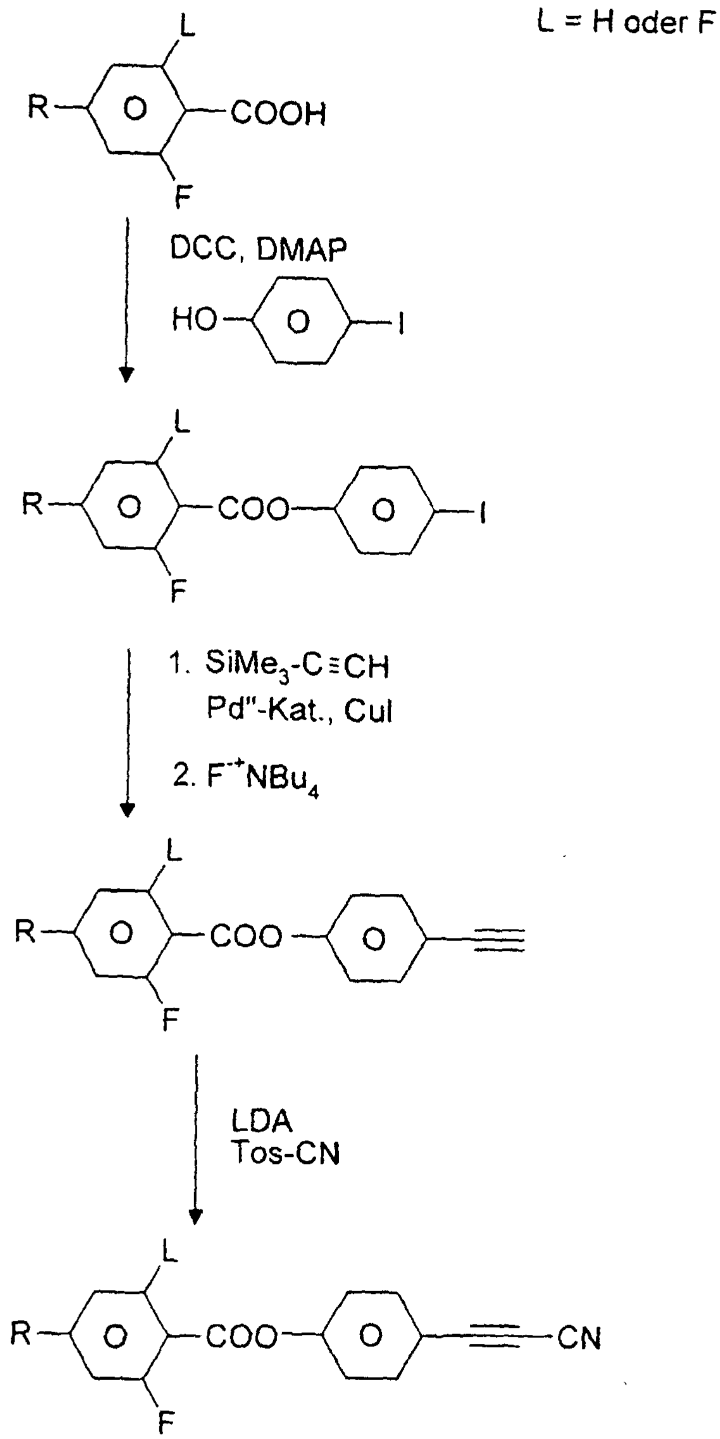

Schema 7

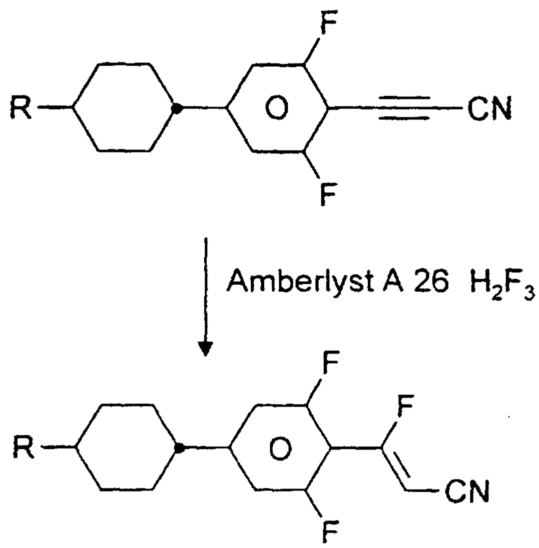


Schema 8

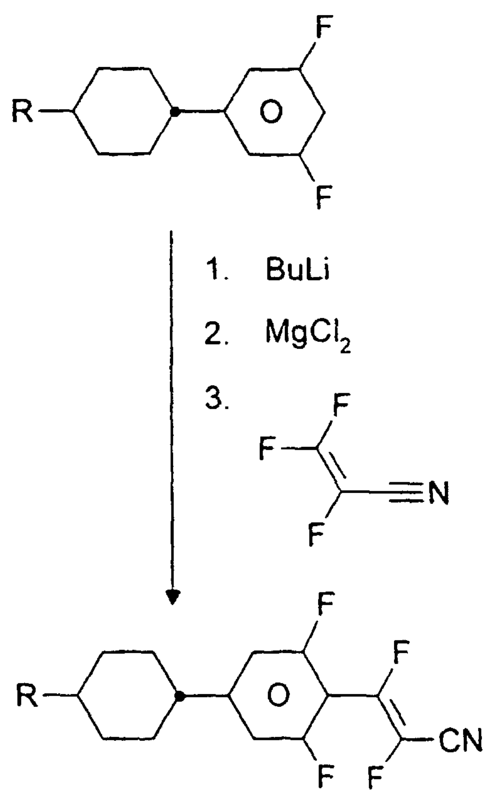

Schema 9

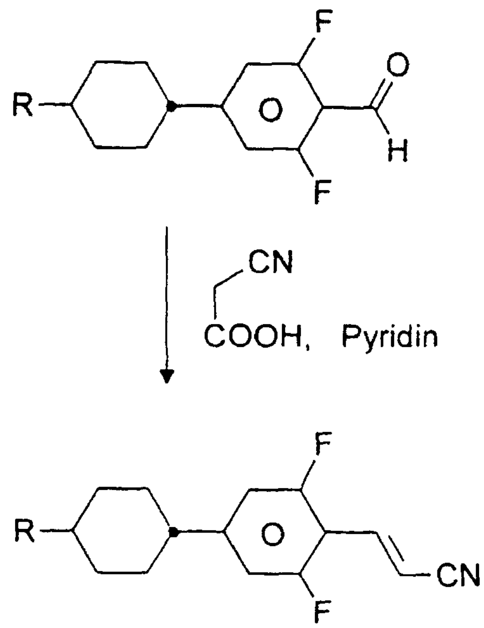

Schema 10

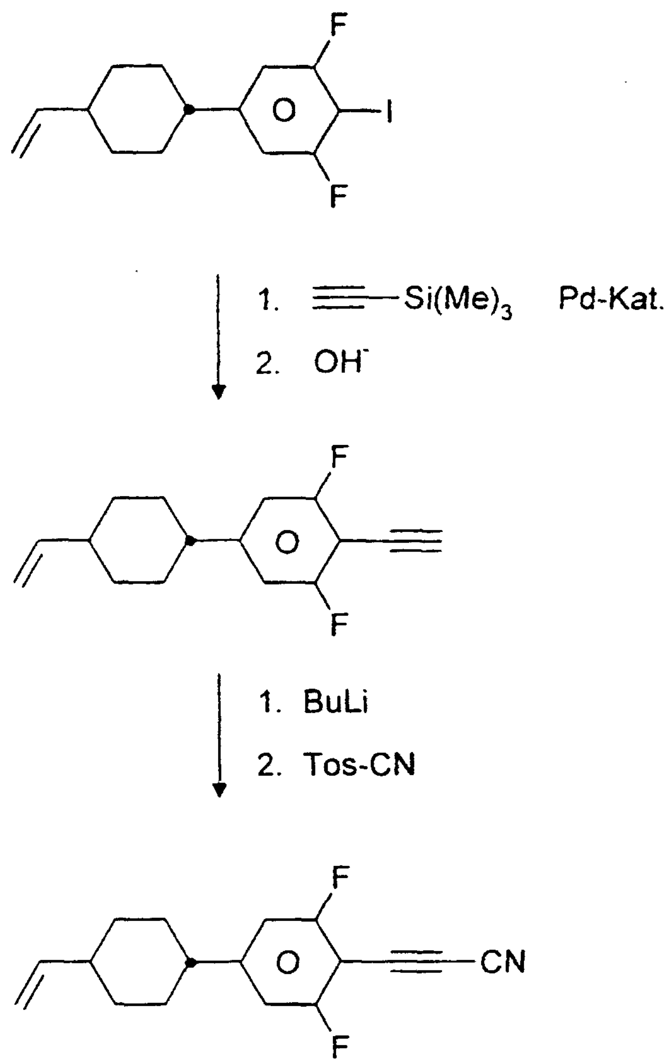


Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexyl-ester, Phenyl- oder Cyclohexyl-ester der Cyclohexylbenzoesäure, Phenyl- oder Cyclohexyl-ester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)-ethane, 1-Cyclohexyl-2-biphenylylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-$CH_2CH_2$-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F. -Cl, -NCS oder $-(O)_iCH_{3-(k+l)}F_kCl_l$, wobei i 0 oder 1 und k+l 1, 2 oder 3 sind; die Verbindungen, in denen R" diese Bedeutung hat, werden mit den Teilformeln 1 b, 2b, 3b, 4b und 5b bezeichnet. Besonders bevorzugt sind solche Verbindungen der Teilformeln 1b, 2b, 3b, 4b und 5b, in denen R" die Bedeutung -F, -Cl, -NCS, $-CF_3$, $-OCHF_2$ oder $-OCF_3$ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN; diese Untergruppe wird im folgenden als Gruppe C bezeichnet und die Verbindungen dieser Untergruppe werden entsprechend mit Teilformeln 1 c, 2c, 3c, 4c und 5 c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise

Gruppe A: 0 bis 90 %, vorzugsweise 20 bis 90 %, insbesondere 30 bis 90 %
Gruppe B: 0 bis 80 %, vorzugsweise 10 bis 80 %, insbesondere 10 bis 65 %
Gruppe C: 0 bis 80 %, vorzugsweise 5 bis 80 %, insbesondere 5 bis 50 %

wobei die Summe der Massenanteile der in den jeweiligen erfindungsgemäßen Medien enthaltenen Verbindungen aus den Gruppen A und/oder B und/oder C vorzugsweise 5 bis 90 % und insbesondere 10 bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste $C_nH_{2n+1}$ und $C_mH_{2m+1}$ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten $R^1$, $R^2$, $L^1$ und $L^2$:

| Code für $R^1$, $R^2$, $L^1$, $L^2$ | $R^1$ | $R_2$ | $L^1$ | $L^2$ |
|---|---|---|---|---|
| nm | $C_nH_{2n+1}$ | $C_mH_{2m+1}$ | H | H |
| nOm | $C_nH_{2n+1}$ | $OC_mH_{2m+1}$ | H | H |
| nO.m | $OC_nH_{2n+1}$ | $C_mH_{2m+1}$ | H | H |
| n | $C_nH_{2n+1}$ | CN | H | H |
| nN.F | $C_nH_{2n+1}$ | CN | H | F |
| nF | $C_nH_{2n+1}$ | F | H | H |
| nOF | $OC_nH_{2n+1}$ | F | H | H |
| nCl | $C_nH_{2n+1}$ | Cl | H | H |
| nF.F | $C_nH_{2n+1}$ | F | H | F |
| nF.F.F | $C_nH_{2n+1}$ | F | F | F |
| $nCF_3$ | $C_nH_{2n+1}$ | $CF_3$ | H | H |
| $nOCF_3$ | $C_nH_{2n+1}$ | $OCF_3$ | H | H |
| $nOCF_2$ | $C_nH_{2n+1}$ | $OCHF_2$ | H | H |
| nS | $C_nH_{2n+1}$ | NCS | H | H |
| rVsN | $C_rH_{2r+1}$-CH=CH-$C_sH_{2s}$- | CN | H | H |
| rEsN | $C_rH_{2r+1}$-O-$C_sH_{2s}$- | CN | H | H |
| nAm | $C_nH_{2n+1}$ | $COOC_mH_{2m+1}$ | H | H |

Gegenstand der Erfindung sind ebenfalls flüssigkristalline Mischungen enthaltend mindestens eine Komponente der Formel I und vorzugsweise mindestens eine weitere Komponente ausgewählt aus den Tabellen A und B.

**Tabelle A:**

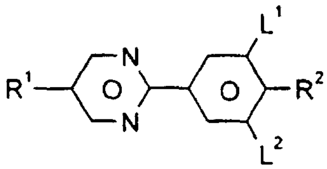

**PYP**

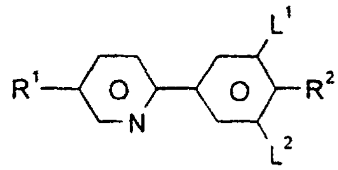

**PYRP**

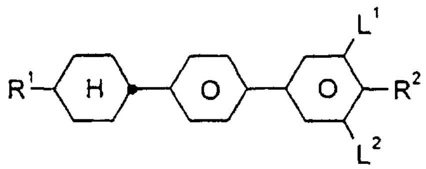

**BCH**

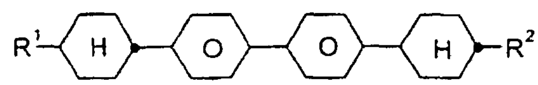

**CBC**

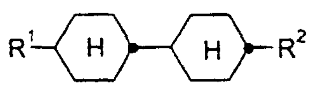

**CCH**

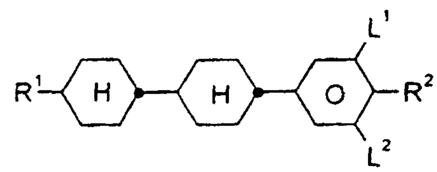

**CCP**

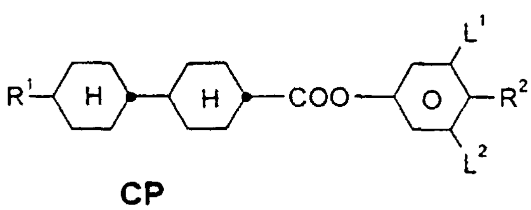

**CP**

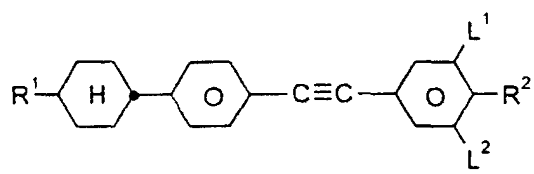

**CPTP**

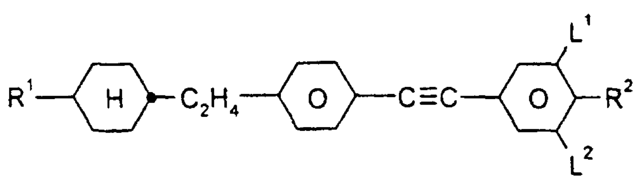

**CEPTP**

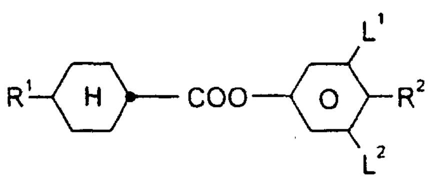

**D**

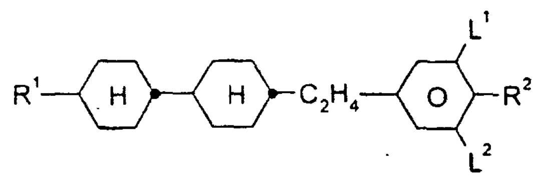

**ECCP**

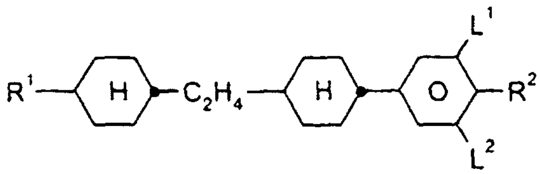

CECP

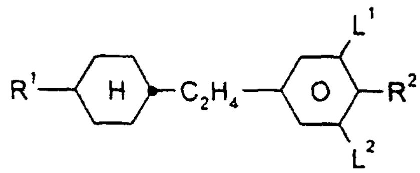

EPCH

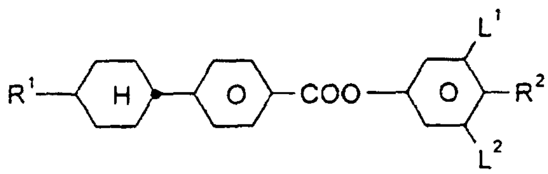

HP

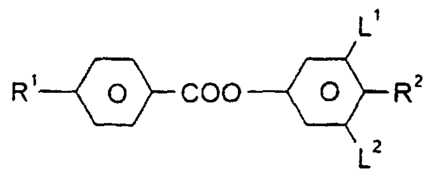

ME

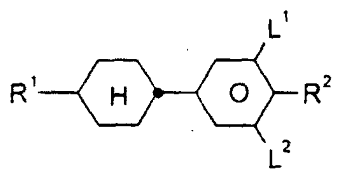

PCH

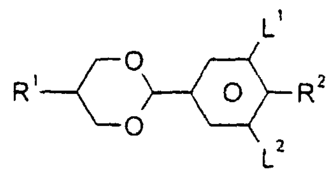

PDX

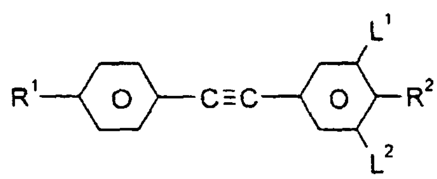

PTP

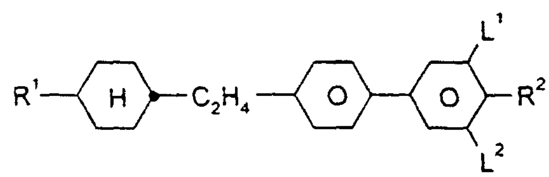

BECH

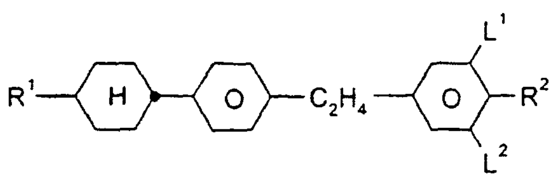

EBCH

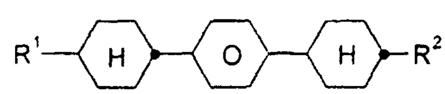

CPC

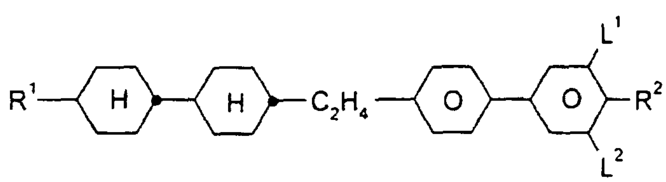

CCEB

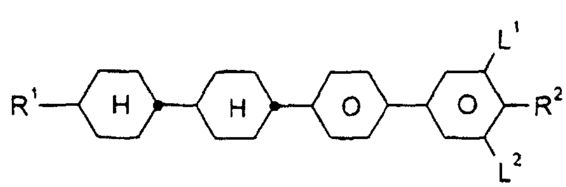

CCB

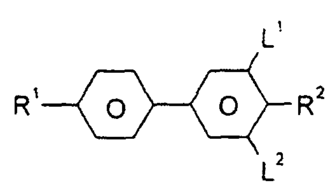

B

## Tabelle B:

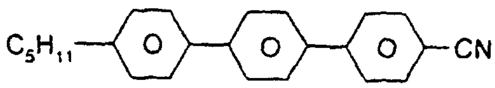

T15

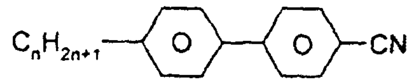

K3n

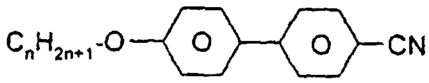

M3n

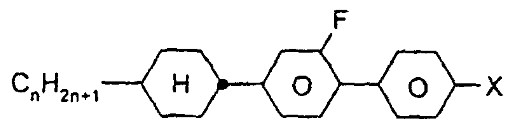

BCH-n.FX

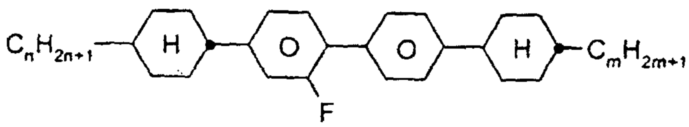

CBC-nmF

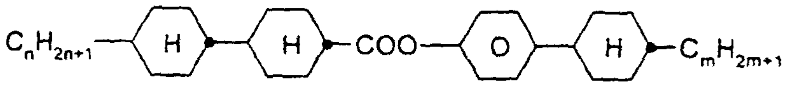

CCPC-nm

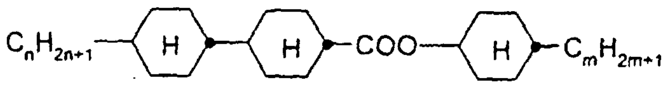

CH-nm

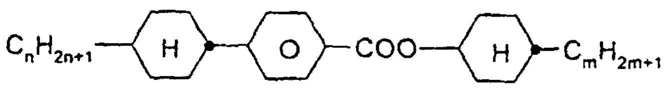

HH-nm

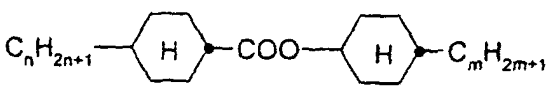

OS-nm

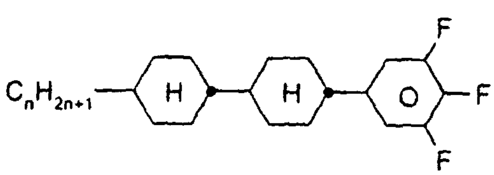

CCP-nF.F.F

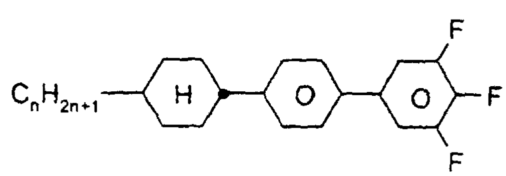

BCH-nF.F.F

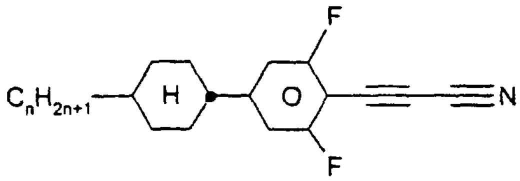

**CU-n-AN**

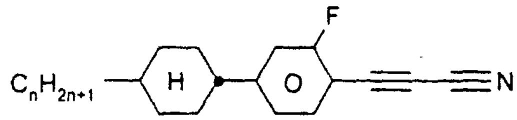

**CG-n-AN**

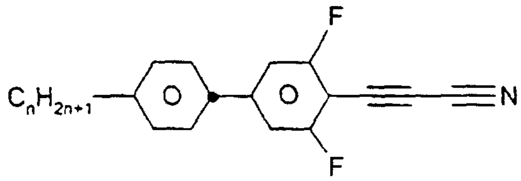

**PU-3-AN**

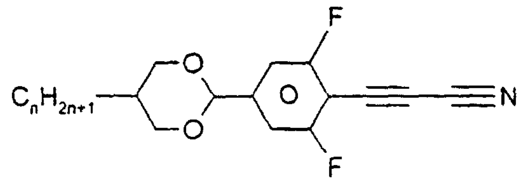

**DU-n-AN**

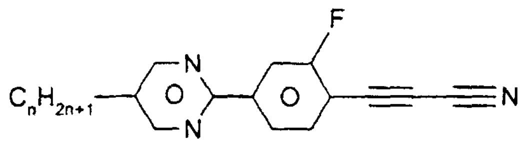

**MG-n-AN**

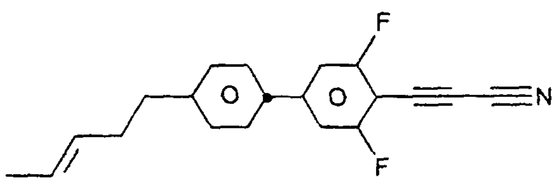

**PU-1V2-AN**

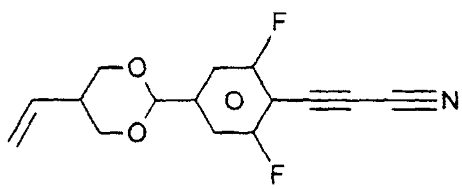

**DU-V-AN**

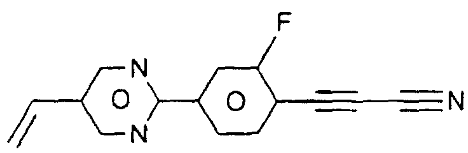

**MG-1V-AN**

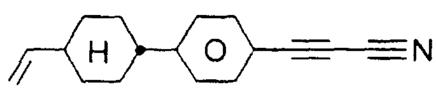

**CP-V-AN**

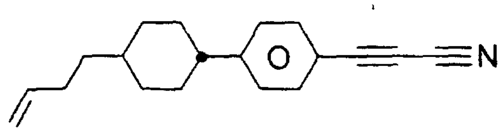

**CP-V2-AN**

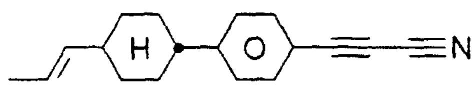

**CP-1V-AN**

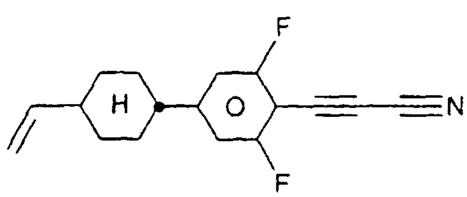

**CU-V-AN**

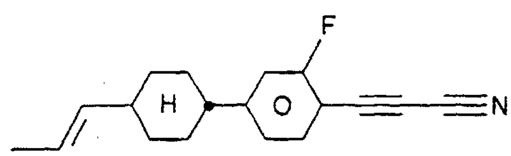

**CG-1V-AN**

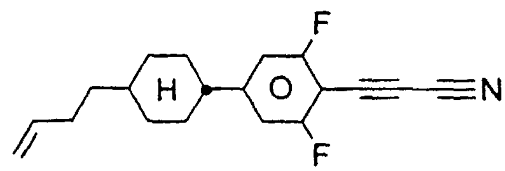

**CU-V2-AN**

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. $\Delta n$ bedeutet optische Anisotropie (589 nm, 20 °C), $\Delta\varepsilon$ die dielektrische Anisotropie [20 °C, 1kHz] und die Fließviskosität ($mm^2$/sec) wurde bei 20 °C bestimmt.

"Übliche Aufarbeitung" bedeutet: man gibt gegebenenfalls Wasser hinzu, extrahiert mit Dichlormethan, Diethylether, Methyl-tert.Butylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:

| | |
|---|---|
| DAST | Diethylaminoschwefeltrifluorid |
| DMEU | 1,3-Dimethyl-2-imidazolidinon |
| KOT | Kalium-tertiär-butanolat |
| THF | Tetrahydrofuran |
| pTsOH | p-Toluolsulfonsäure |

**Beispiel 1**

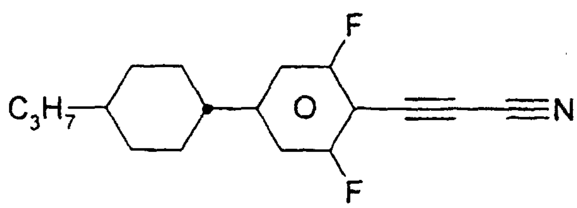

Schritt 1.1

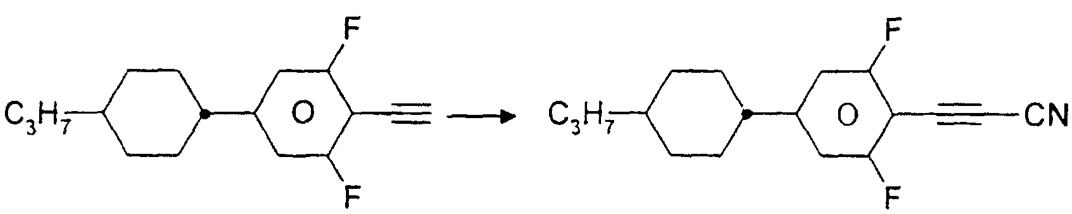

A B

In einer Stickstoffatmosphäre werden unter Rühren 0,019 mol A zu 30 ml abs. THF zugegeben. Bei -65 °C wird zu dem Gemisch 0,021 mol BuLi (15%ige Lösung in n-Hexan) zugetropft und anschließend wird 0,5 h gerührt. Nach Zugabe von 0,021 mol p-Toluolsulfonylcyanid in 10 ml THF bei -60 °C läßt man das Gemisch auf 5 °C erwärmen. Nach der Hydrolyse wird die Lösung wie üblich aufgearbeitet. Das Rohprodukt wird aus Ethanol und n-Heptan umkristallisiert. K 68 N (61,3) I; $\Delta\varepsilon$ = 42,25; $\Delta n$ = + 0,208

Analog werden die folgenden Verbindungen der Formel

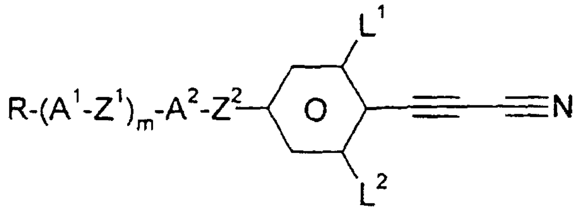

hergestellt:

| R | $-(A^1-Z^1)_m-A^2-Z^2-$ | $L^1$ | $L^2$ | |
|---|---|---|---|---|
| $C_2H_5$ | 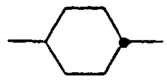 | F | H | K 39 N 46,4 I; Δn = +0,202; Δε = 36,01 |
| $C_2H_5$ | 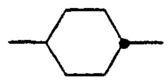 | F | F | K 45 I; Δn = 0,179; Δε = 40,45 |
| $n-C_3H_7$ | 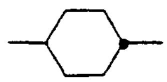 | F | H | K 49 N 90,8 I; Δn = +0,225; Δε = 35,92 |
| $n-C_4H_9$ | 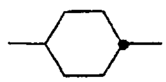 | F | H | K 18 N 91,3 I; Δn = +0,214; Δε = 33,59 |
| $n-C_4H_9$ | 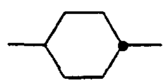 | F | F | K 34 N 59,9 I; Δn = +0,182; Δε = 39,57 |
| $n-C_5H_{11}$ | 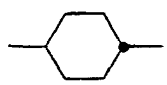 | F | H | K 35 N 101 I; Δn = +0,221; Δε = 32,94 |
| $n-C_5H_{11}$ | 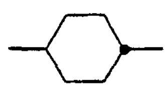 | F | F | K 53 N 72,5 I; Δn = +0,194; Δε = 38,6 |
| $n-C_6H_{13}$ | 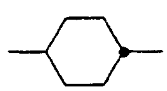 | F | H | |
| $n-C_6H_{13}$ | 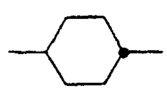 | F | F | |

| R | $-(A^1-Z^1)_m-A^2-Z^2-$ | $L^1$ | $L^2$ | |
|---|---|---|---|---|
| $C_2H_5$ | 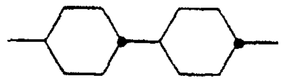 | F | F | K 96 N 268 I; $\Delta n$ = +0,237; $\Delta\varepsilon$ = 37,02 |
| n-$C_3H_7$ | 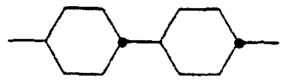 | F | F | |
| n-$C_5H_{11}$ | 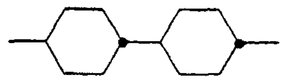 | F | F | |
| $C_2H_5$ | 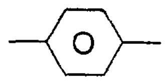 | F | H | |
| $C_2H_5O$ | 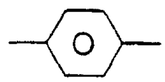 | F | H | |
| $C_2H_5$ | 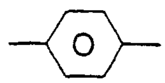 | F | F | |
| $C_2H_5O$ | 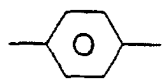 | F | F | |
| n-$C_3H_7$ | 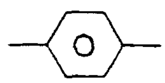 | F | H | |
| n-$C_3H_7$ | 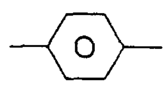 | F | F | K 75 N (51,0) I; $\Delta n$ = +0,330; $\Delta\varepsilon$ = 51,48 |
| n-$C_3H_7O$ | 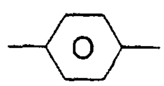 | F | F | |
| n-$C_4H_9$ | 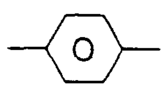 | F | H | |
| n-$C_4H_9$ | 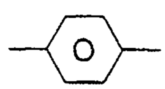 | F | F | |
| n-$C_5H_{11}$ | 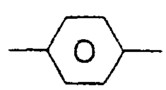 | F | H | |

| R | $-(A^1-Z^1)_m-A^2-Z^2-$ | $L^1$ | $L^2$ | |
|---|---|---|---|---|
| $n-C_5H_{11}$ | 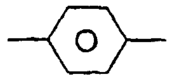 | F | F | K 61 N (48,8) I; $\Delta n = +0{,}320$; $\Delta\varepsilon = 45{,}87$ |
| $C_2H_5$ | 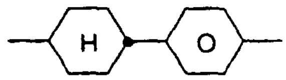 | F | H | |
| $C_2H_5$ | 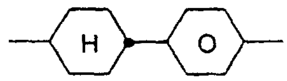 | F | F | |
| $n-C_3H_7$ | 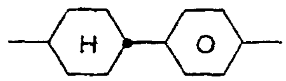 | F | H | |
| $n-C_3H_7$ | 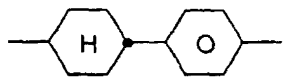 | F | F | |
| $n-C_5H_{11}$ | 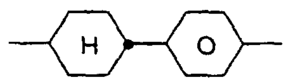 | F | H | |
| $n-C_5H_{11}$ | 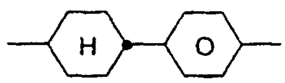 | F | F | |
| $n-C_6H_{13}$ | 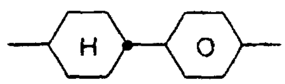 | F | H | |
| $n-C_6H_{13}$ | 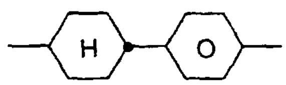 | F | F | |
| $C_2H_5$ | 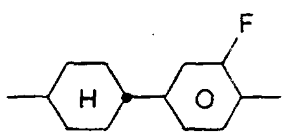 | F | H | |
| $C_2H_5$ | 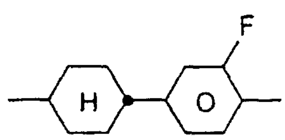 | F | F | |
| $n-C_3H_7$ | 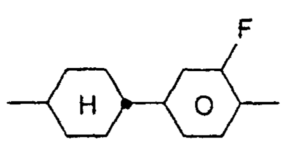 | F | H | |
| $n-C_3H_7$ | 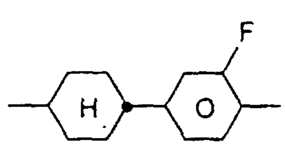 | F | F | |

| R | $-(A^1-Z^1)_m-A^2-Z^2-$ | $L^1$ | $L^2$ |
|---|---|---|---|
| $n-C_5H_{11}$ | 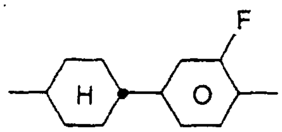 | F | H |
| $n-C_5H_{11}$ | 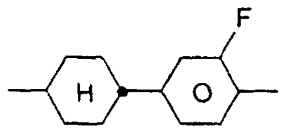 | F | F |
| $n-C_6H_{13}$ | 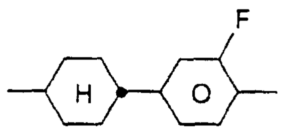 | F | H |
| $n-C_6H_{13}$ | 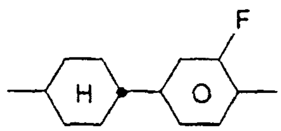 | F | F |
| $C_2H_5$ | 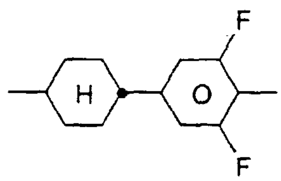 | F | H |
| $C_2H_5$ | 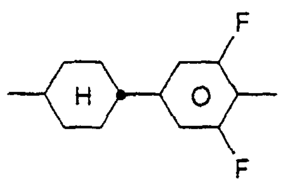 | F | F |
| $n-C_3H_7$ | 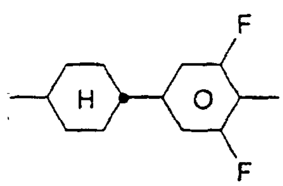 | F | H |
| $n-C_3H_7$ | 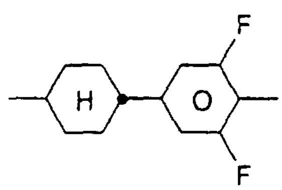 | F | F |
| $n-C_5H_{11}$ | 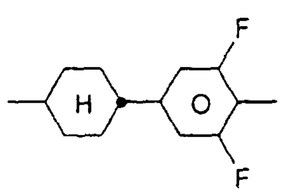 | F | H |

| R | $-(A^1-Z^1)_m-A^2-Z^2-$ | $L^1$ | $L^2$ | |
|---|---|---|---|---|
| $n-C_5H_{11}$ | 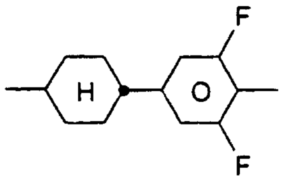 | F | F | |
| $n-C_3H_7$ | 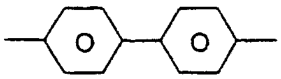 | F | H | |
| $n-C_3H_7O$ | 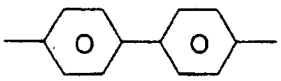 | F | H | |
| $n-C_3H_7$ | 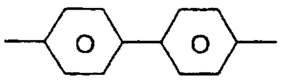 | F | F | |
| $n-C_3H_7O$ | 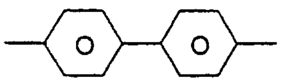 | F | F | |
| $n-C_5H_{11}$ | 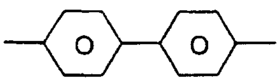 | F | H | |
| $n-C_5H_{11}O$ | 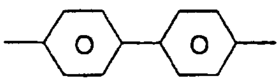 | F | H | |
| $n-C_3H_7$ | 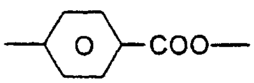 | H | H | K 84 N 139 I; $\Delta n$ = +0,266; $\Delta\varepsilon$ = 44,88 |
| $n-C_3H_7$ | 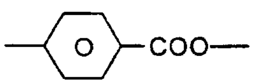 | H | F | |
| $n-C_5H_{11}$ | 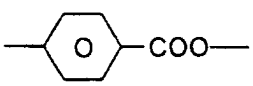 | H | H | |
| $n-C_5H_{11}$ | 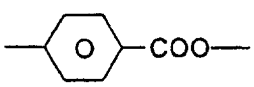 | H | F | |
| $n-C_5H_{11}$ | 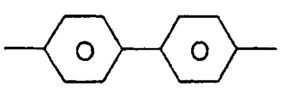 | F | F | |
| $n-C_5H_{11}O$ | 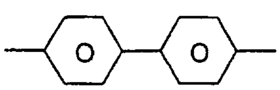 | F | F | |
| $C_2H_5$ | 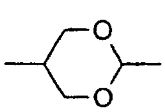 | F | H | |

| R | $-(A^1-Z^1)_m-A^2-Z^2-$ | $L^1$ | $L^2$ | |
|---|---|---|---|---|
| $C_2H_5$ | 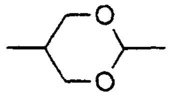 | F | F | |
| $n-C_3H_7$ | 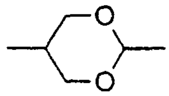 | F | H | |
| $n-C_3H_7$ | 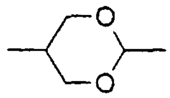 | F | F | K 71 I;<br>$\Delta n$ = +0,175;<br>$\Delta\varepsilon$ = 59,78 |
| $n-C_5H_{11}$ | 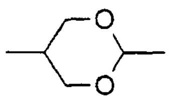 | F | H | |
| $n-C_5H_{11}$ | 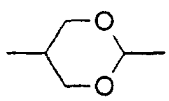 | F | F | |
| $n-C_3H_7$ | 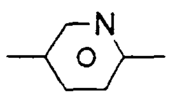 | F | F | |
| $n-C_3H_7$ | 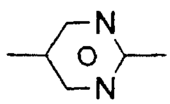 | F | H | |
| $n-C_3H_7O$ | 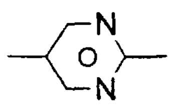 | F | H | |
| $n-C_3H_7$ | 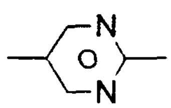 | F | F | K 136 I |
| $n-C_3H_7O$ | 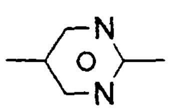 | F | F | |
| $n-C_5H_{11}$ | 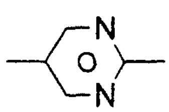 | F | H | K 79 N 88,9 I;<br>$\Delta n$ = +0,315;<br>$\Delta\varepsilon$ = 57,02 |
| $n-C_3H_7$ | 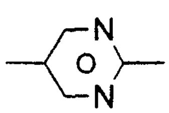 | H | H | K 143 N (141,6) I; |

| R | $-(A^1-Z^1)_m-A^2-Z^2-$ | $L^1$ | $L^2$ | |
|---|---|---|---|---|
| $n-C_5H_{11}O$ | 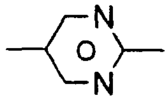 | F | H | |
| $n-C_5H_{11}$ | 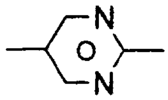 | F | F | |
| $n-C_5H_{11}O$ | 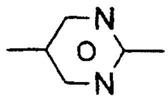 | F | F | |
| $C_2H_5$ | 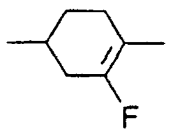 | F | H | |
| $C_2H_5$ | 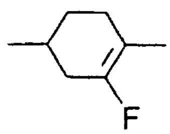 | F | F | |
| $n-C_3H_7$ | 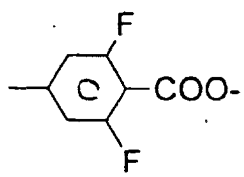 | H | H | K 69 N 92,4 I; $\Delta n$ = +0,249; $\Delta\varepsilon$ = 46,7 |
| $n-C_5H_{11}$ | 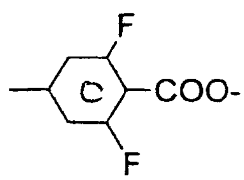 | H | F | |

**Beispiel 2**

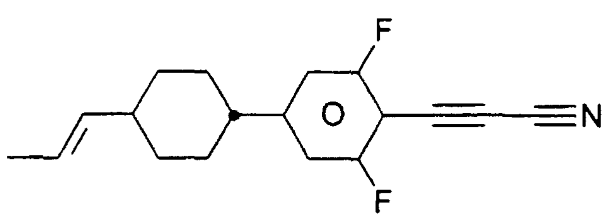

Schritt 2.1

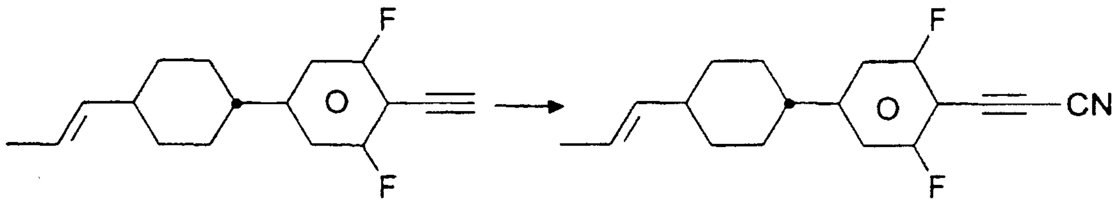

A

B

In einer Stickstoffatmosphäre werden unter Rühren 0,019 mol A zu 30 ml abs. THF zugegeben. Bei -65 °C wird zu dem Gemisch 0,021 mol BuLi (15%ige Lösung in n-Hexan) zugetropft und anschließend wird 0,5 h gerührt. Nach Zugabe von 0,021 mol p-Toluolsulfonylcyanid in 10 ml THF bei -60 °C läßt man das Gemisch auf 5 °C erwärmen. Nach der Hydrolyse wird die Lösung wie üblich aufgearbeitet. Das Rohprodukt wird aus Ethanol und n-Heptan umkristallisiert.

Analog werden die folgenden Verbindungen der Formel

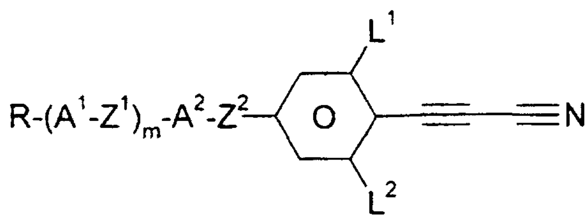

hergestellt:

| R | $-(A^1-Z^1)_m-A^2-Z^2-$ | $L^1$ | $L^2$ | |
|---|---|---|---|---|
| $CH_2=CH$ | 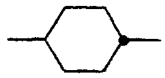 | H | H | K 65 N 120,4 I; $\Delta n$ = +0,279; $\Delta\varepsilon$ = 28,77 |
| $CH_2=CH$ | 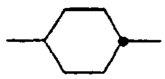 | H | F | |
| $CH_2=CH$ | 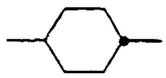 | F | F | K 69 N (49,5) I; $\Delta n$ = +0,220; $\Delta\varepsilon$ = 39,1 |
| $CH_3CH=CH$ | 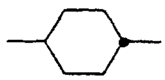 | H | H | K 99 N 172,5 I; $\Delta n$ = +0,295; $\Delta\varepsilon$ = 35,1 |
| $CH_3CH=CH$ | 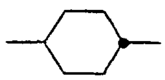 | H | F | |
| $C_2H_5CH=CH$ | 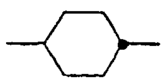 | H | H | |
| $C_2H_5CH=CH$ | 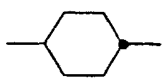 | H | F | |
| $C_2H_5CH=CH$ | 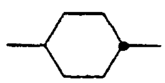 | F | F | |
| $C_3H_7CH=CH$ | 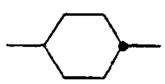 | H | H | |
| $C_3H_7CH=CH$ | 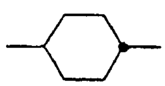 | H | F | |
| $C_3H_7CH=CH$ | 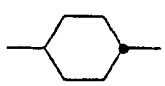 | F | F | |
| $CH_2=CHCH_2CH_2$ | 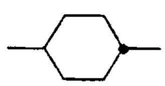 | H | H | K 65 N 133,5 I; $\Delta n$ = +0,265; $\Delta\varepsilon$ = 27,92 |
| $CH_2=CHCH_2CH_2$ | 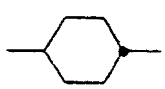 | H | F | |
| $CH_2=CHCH_2CH_2$ | 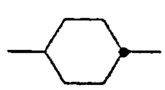 | F | F | K 57 N 78,9 I; $\Delta n$ = +0,209; $\Delta\varepsilon$ = 35,3 |

| R | $-(A^1-Z^1)_m-A^2-Z^2-$ | $L^1$ | $L^2$ |
|---|---|---|---|
| $CH_3CH=CHCH_2CH_2$ | 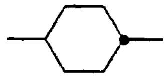 | H | H |
| $CH_3CH=CHCH_2CH_2$ | 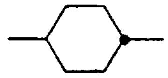 | H | F |
| $CH_3CH=CHCH_2CH_2$ | 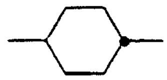 | F | F |
| $C_2H_5CH=CHCH_2CH_2$ | 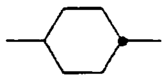 | H | H |
| $C_2H_5CH=CHCH_2CH_2$ | 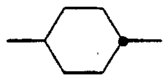 | H | F |
| $C_2H_5CH=CHCH_2CH_2$ | 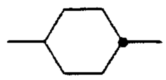 | F | F |
| $C_3H_7CH=CHCH_2CH_2$ | 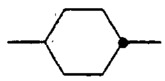 | H | H |
| $C_3H_7CH=CHCH_2CH_2$ | 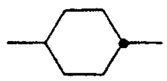 | H | F |
| $C_3H_7CH=CHCH_2CH_2$ | 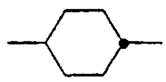 | F | F |
| $CH_2=CH-$ | 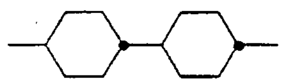 | H | F |
| $CH_3CH=CH-$ | 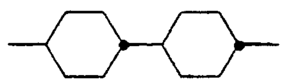 | H | F |
| $C_2H_5CH=CH-$ | 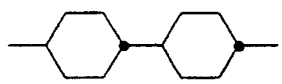 | H | F |
| $C_3H_7-CH=CH-$ | 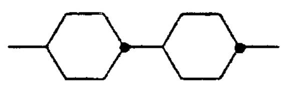 | H | F |
| $CH_2=CH$ | 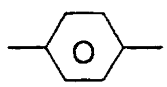 | H | H |
| $CH_2=CH-O-$ | 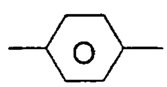 | H | H |
| $CH_2=CH$ | 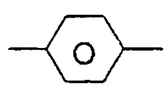 | H | F |
| $CH_2=CH-O-$ | 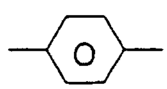 | H | F |
| $CH_2=CH$ | 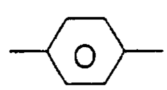 | F | F |

| R | $-(A^1-Z^1)_m-A^2-Z^2-$ | $L^1$ | $L^2$ |
|---|---|---|---|
| $CH_2=CH-O-$ | 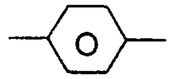 | F | F |
| $CH_3CH=CH$ | 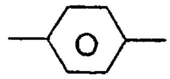 | H | H |
| $CH_3CH=CH$ | 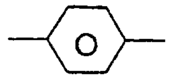 | H | F |
| $CH_3CH=CH$ | 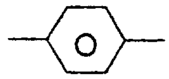 | F | F |
| $C_2H_5CH=CH$ | 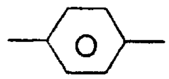 | H | H |
| $C_2H_5CH=CH$ | 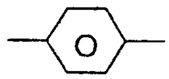 | H | F |
| $C_2H_5CH=CH$ | 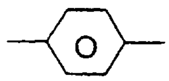 | F | F |
| $C_3H_7CH=CH$ | 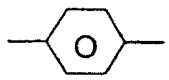 | H | H |
| $C_3H_7CH=CH$ | 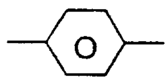 | H | F |
| $C_3H_7CH=CH$ | 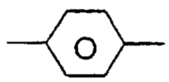 | F | F |
| $CH_2=CHCH_2CH_2$ | 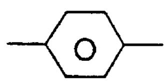 | H | H |
| $CH_2=CHCH_2CH_2$ | 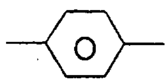 | H | F |
| $CH_2=CHCH_2CH_2$ | 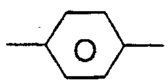 | F | F |
| $CH_3CH=CHCH_2CH_2$ | 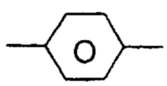 | H | H |
| $CH_3CH=CHCH_2CH_2$ | 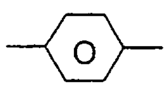 | H | F |
| $CH_3CH=CHCH_2CH_2$ | 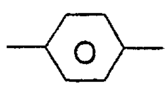 | F | F |
| $C_2H_5CH=CHCH_2CH_2$ | 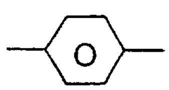 | H | H |
| $C_2H_5CH=CHCH_2CH_2$ | 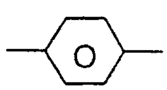 | H | F |

| R | $-(A^1-Z^1)_m-A^2-Z^2-$ | $L^1$ | $L^2$ |
|---|---|---|---|
| $C_2H_5CH=CHCH_2CH_2$ | 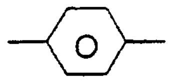 | F | F |
| $C_3H_7CH=CHCH_2CH_2$ | 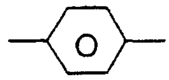 | H | H |
| $C_3H_7CH=CHCH_2CH_2$ | 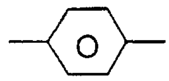 | H | F |
| $C_3H_7CH=CHCH_2CH_2$ | 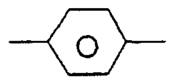 | F | F |
| $CH_2=CH$ | 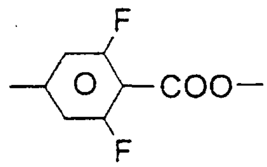 | H | H |
| $CH_2=CH$ | 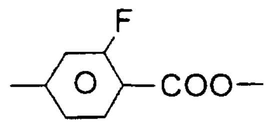 | H | H |
| $CH_3-CH=CH$ | 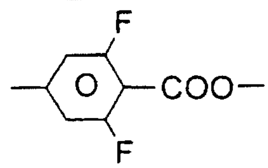 | H | H |
| $CH_3-CH=CH$ | 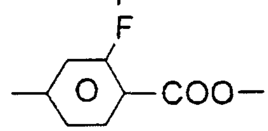 | H | H |
| $CH_2=CH$ | 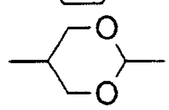 | H | H |
| $CH_2=CH$ | 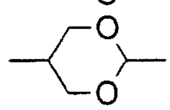 | H | F |
| $CH_2=CH$ | 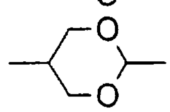 | F | F |
| $CH_3CH=CH$ | 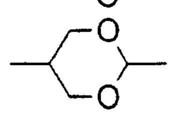 | H | H |
| $CH_3CH=CH$ | 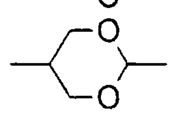 | H | F |
| $CH_3CH=CH$ | 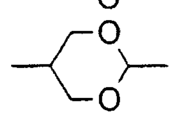 | F | F |

| R | $-(A^1-Z^1)_m-A^2-Z^2-$ | $L^1$ | $L^2$ |
|---|---|---|---|
| $C_2H_5CH=CH$ | 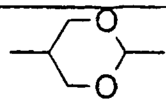 | H | H |
| $C_2H_5CH=CH$ | 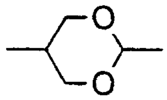 | H | F |
| $C_2H_5CH=CH$ | 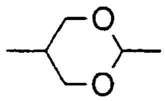 | F | F |
| $C_3H_7CH=CH$ | 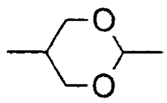 | H | H |
| $C_3H_7CH=CH$ | 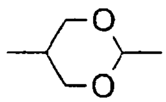 | H | F |
| $C_3H_7CH=CH$ | 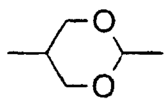 | F | F |
| $CH_2=CHCH_2CH_2$ | 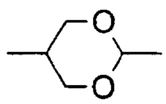 | H | H |
| $CH_2=CHCH_2CH_2$ | 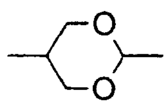 | H | F |
| $CH_2=CHCH_2CH_2$ | 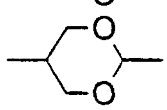 | F | F |
| $CH_3CH=CHCH_2CH_2$ | 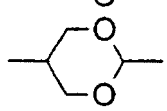 | H | H |
| $CH_3CH=CHCH_2CH_2$ | 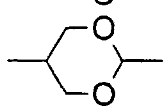 | H | F |
| $CH_3CH=CHCH_2CH_2$ | 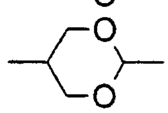 | F | F |
| $C_2H_5CH=CHCH_2CH_2$ | 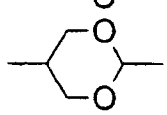 | H | H |
| $C_2H_5CH=CHCH_2CH_2$ | 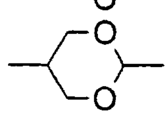 | H | F |
| $C_2H_5CH=CHCH_2CH_2$ | 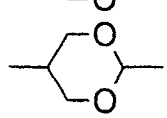 | F | F |
| $C_3H_7CH=CHCH_2CH_2$ | 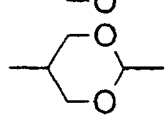 | H | H |

| R | $-(A^1-Z^1)_m-A^2-Z^2-$ | $L^1$ | $L^2$ |
|---|---|---|---|
| $C_3H_7CH=CHCH_2CH_2$ | 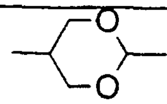 | H | F |
| $C_3H_7CH=CHCH_2CH_2$ | 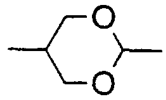 | F | F |
| $CH_2=CH$ | 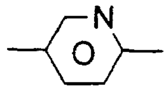 | H | H |
| $CH_2=CH$ | 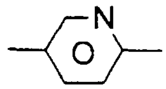 | H | F |
| $CH_2=CH$ | 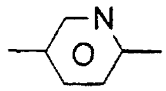 | F | F |
| $CH_3CH=CH$ | 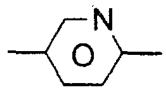 | H | H |
| $CH_3CH=CH$ | 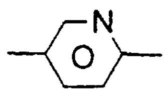 | H | F |
| $CH_3CH=CH$ | 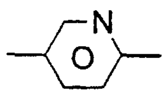 | F | F |
| $C_2H_5CH=CH$ | 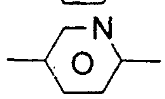 | H | H |
| $C_2H_5CH=CH$ | 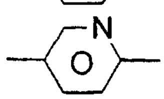 | H | F |
| $C_2H_5CH=CH$ | 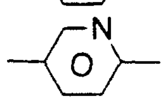 | F | F |
| $C_3H_7CH=CH$ | 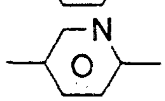 | H | H |
| $C_3H_7CH=CH$ | 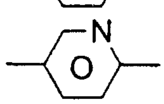 | H | F |
| $C_3H_7CH=CH$ | 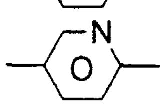 | F | F |
| $CH_2=CHCH_2CH_2$ | 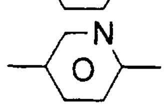 | H | H |
| $CH_2=CHCH_2CH_2$ | 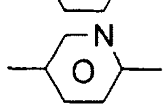 | H | F |
| $CH_2=CHCH_2CH_2$ | 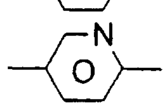 | F | F |

| R | $-(A^1-Z^1)_m-A^2-Z^2-$ | $L^1$ | $L^2$ |
|---|---|---|---|
| $CH_3CH=CHCH_2CH_2$ | 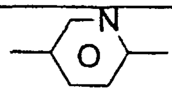 | H | H |
| $CH_3CH=CHCH_2CH_2$ | 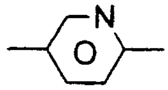 | H | F |
| $CH_3CH=CHCH_2CH_2$ | 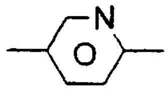 | F | F |
| $C_2H_5CH=CHCH_2CH_2$ | 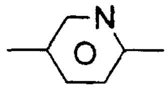 | H | H |
| $C_2H_5CH=CHCH_2CH_2$ | 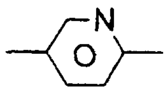 | H | F |
| $C_2H_5CH=CHCH_2CH_2$ | 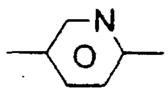 | F | F |
| $C_3H_7CH=CHCH_2CH_2$ | 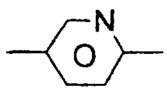 | H | H |
| $C_3H_7CH=CHCH_2CH_2$ | 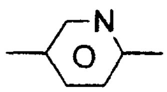 | H | F |
| $C_3H_7CH=CHCH_2CH_2$ | 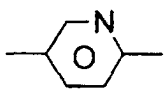 | F | F |
| $CH_2=CH$ | 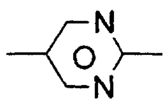 | H | H |
| $CH_2=CH$ | 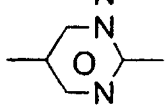 | H | F |
| $CH_2=CH$ | 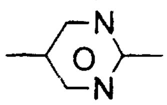 | F | F |
| $CH_3CH=CH$ | 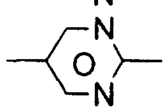 | H | H |
| $CH_3CH=CH$ | 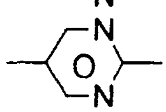 | H | F |
| $CH_3CH=CH$ | 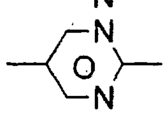 | F | F |
| $C_2H_5CH=CH$ | 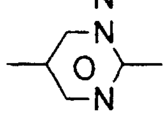 | H | H |
| $C_2H_5CH=CH$ | 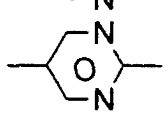 | H | F |

| R | $-(A^1-Z^1)_m-A^2-Z^2-$ | $L^1$ | $L^2$ |
|---|---|---|---|
| $C_2H_5CH=CH$ | 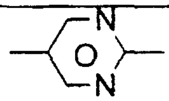 | F | F |
| $C_3H_7CH=CH$ | 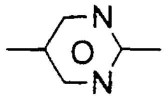 | H | H |
| $C_3H_7CH=CH$ | 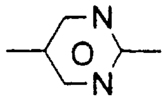 | H | F |
| $C_3H_7CH=CH$ | 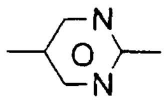 | F | F |
| $CH_2=CHCH_2CH_2$ | 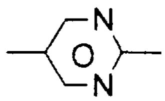 | H | H |
| $CH_2=CHCH_2CH_2$ | 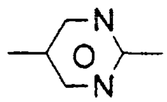 | H | F |
| $CH_2=CHCH_2CH_2$ | 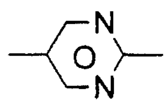 | F | F |
| $CH_3CH=CHCH_2CH_2$ | 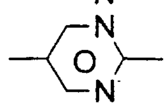 | H | H |
| $CH_3CH=CHCH_2CH_2$ | 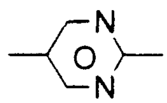 | H | F |
| $CH_3CH=CHCH_2CH_2$ | 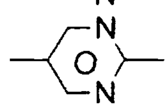 | F | F |
| $C_2H_5CH=CHCH_2CH_2$ | 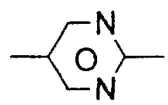 | H | H |
| $C_2H_5CH=CHCH_2CH_2$ | 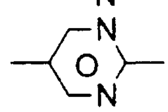 | H | F |
| $C_2H_5CH=CHCH_2CH_2$ | 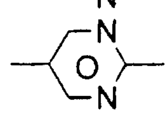 | F | F |
| $C_3H_7CH=CHCH_2CH_2$ | 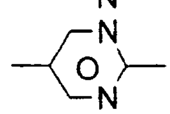 | H | H |
| $C_3H_7CH=CHCH_2CH_2$ | 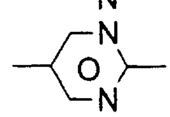 | H | F |
| $C_3H_7CH=CHCH_2CH_2$ | 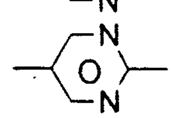 | F | F |

| R | $-(A^1-Z^1)_m-A^2-Z^2-$ | $L^1$ | $L^2$ |
|---|---|---|---|
| $CH_2=CH$ | 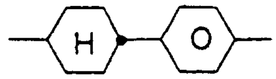 | H | H |
| $CH_2=CH$ | 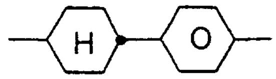 | H | F |
| $CH_2=CH$ | 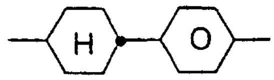 | F | F |
| $CH_3CH=CH$ | 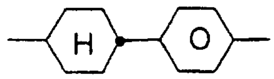 | H | H |
| $CH_3CH=CH$ | 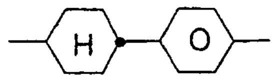 | H | F |
| $CH_3CH=CH$ | 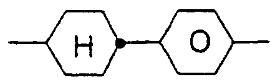 | F | F |
| $C_2H_5CH=CH$ | 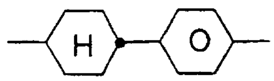 | H | H |
| $C_2H_5CH=CH$ | 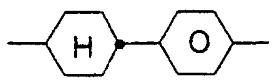 | H | F |
| $C_2H_5CH=CH$ | 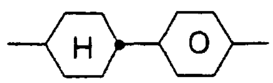 | F | F |
| $C_3H_7CH=CH$ | 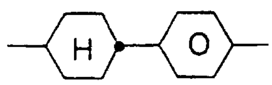 | H | H |
| $C_3H_7CH=CH$ | 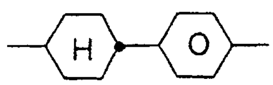 | H | F |
| $C_3H_7CH=CH$ | 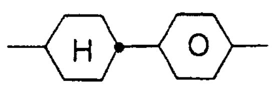 | F | F |
| $CH_2=CHCH_2CH_2$ | 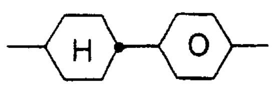 | H | H |
| $CH_2=CHCH_2CH_2$ | 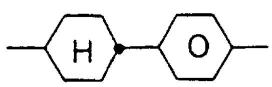 | H | F |
| $CH_2=CHCH_2CH_2$ | 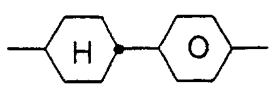 | F | F |
| $CH_3CH=CHCH_2CH_2$ | 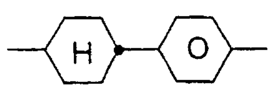 | H | H |
| $CH_3CH=CHCH_2CH_2$ | 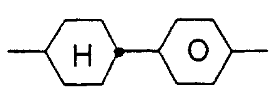 | H | F |
| $CH_3CH=CHCH_2CH_2$ | 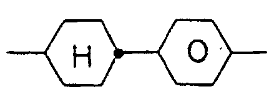 | F | F |

| R | $-(A^1-Z^1)_m-A^2-Z^2-$ | $L^1$ | $L^2$ |
|---|---|---|---|
| $C_2H_5CH=CH_2CH_2$ | 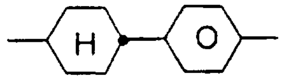 | H | H |
| $C_2H_5CH=CH_2CH_2$ | 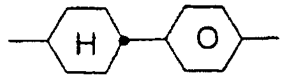 | H | F |
| $C_2H_5CH=CH_2CH_2$ | 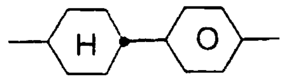 | F | F |
| $C_3H_7CH=CHCH_2CH_2$ | 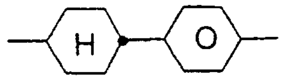 | H | H |
| $C_3H_7CH=CHCH_2CH_2$ | 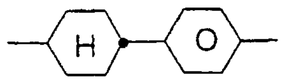 | H | F |
| $C_3H_7CH=CHCH_2CH_2$ | 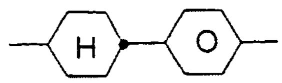 | F | F |
| $CH_2=CH$ | 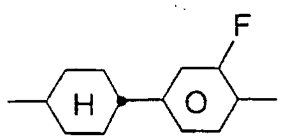 | H | H |
| $CH_2=CH$ | 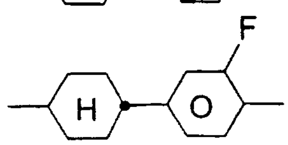 | H | F |
| $CH_2=CH$ | 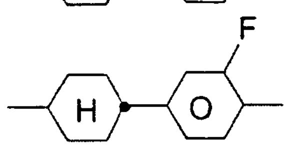 | F | F |
| $CH_3CH=CH$ | 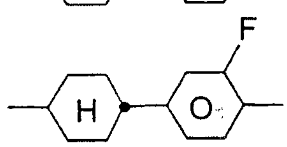 | H | H |
| $CH_3CH=CH$ | 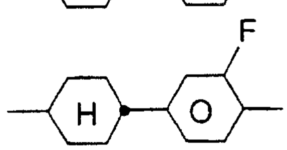 | H | F |
| $CH_3CH=CH$ | 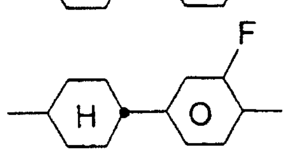 | F | F |
| $C_2H_5CH=CH$ | 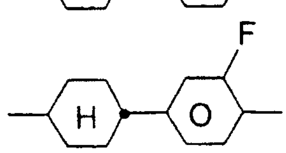 | H | H |

| R | $-(A^1-Z^1)_m-A^2-Z^2-$ | $L^1$ | $L^2$ |
|---|---|---|---|
| $C_2H_5CH=CH$ | 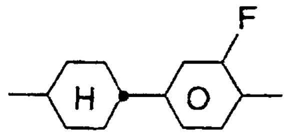 | H | F |
| $C_2H_5CH=CH$ | 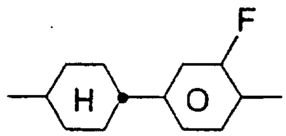 | F | F |
| $C_3H_7CH=CH$ | 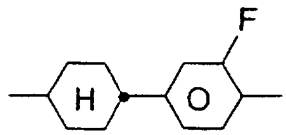 | H | H |
| $C_3H_7CH=CH$ | 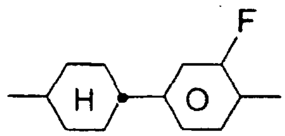 | H | F |
| $C_3H_7CH=CH$ | 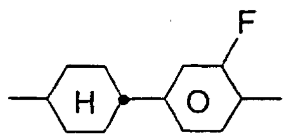 | F | F |
| $CH_2=CHCH_2CH_2$ | 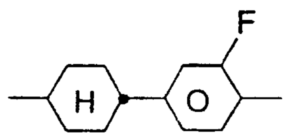 | H | H |
| $CH_2=CHCH_2CH_2$ | 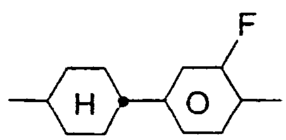 | H | F |
| $CH_2=CHCH_2CH_2$ | 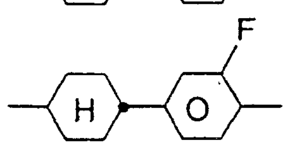 | F | F |
| $CH_3CH=CHCH_2CH_2$ | 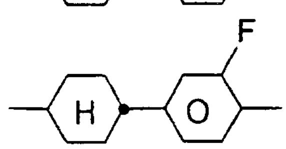 | H | H |
| $CH_3CH=CHCH_2CH_2$ | 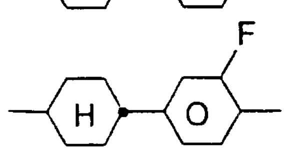 | H | F |
| $CH_3CH=CHCH_2CH_2$ | 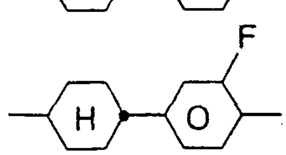 | F | F |

| R | $-(A^1-Z^1)_m-A^2-Z^2-$ | $L^1$ | $L^2$ |
|---|---|---|---|
| $C_2H_5CH{=}CHCH_2CH_2$ | 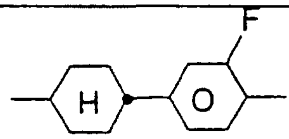 | H | H |
| $C_2H_5CH{=}CHCH_2CH_2$ | 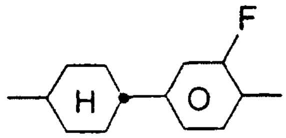 | H | F |
| $C_2H_5CH{=}CHCH_2CH_2$ | 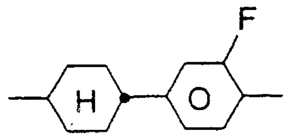 | F | F |
| $C_3H_7CH{=}CHCH_2CH_2$ | 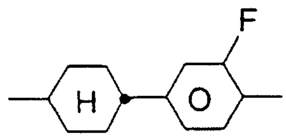 | H | H |
| $C_3H_7CH{=}CHCH_2CH_2$ | 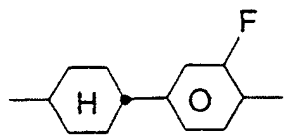 | H | F |
| $C_3H_7CH{=}CHCH_2CH_2$ | 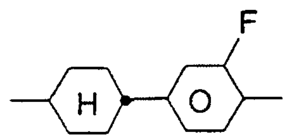 | F | F |
| $CH_2{=}CH$ | 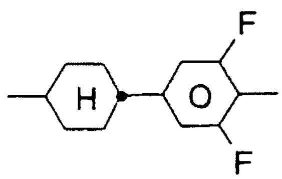 | H | H |
| $CH_2{=}CH$ | 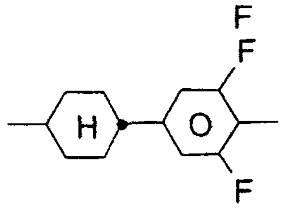 | H | F |
| $CH_2{=}CH$ | 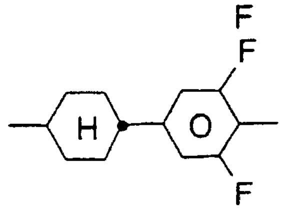 | F | F |
| $CH_3CH{=}CH$ | 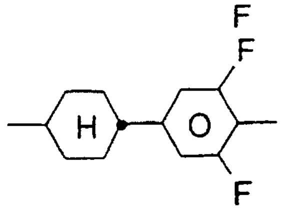 | H | H |

| R | $-(A^1-Z^1)_m-A^2-Z^2-$ | $L^1$ | $L^2$ |
|---|---|---|---|
| $CH_3CH=CH$ | 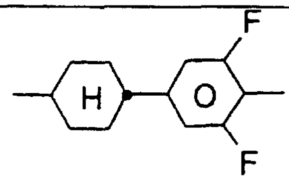 | H | F |
| $CH_3CH=CH$ | 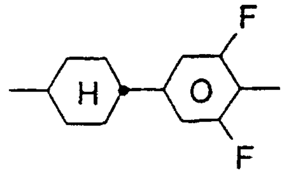 | F | F |
| $C_2H_5CH=CH$ | 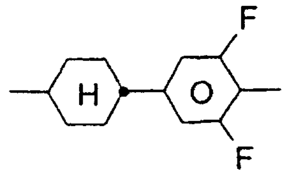 | H | H |
| $C_2H_5CH=CH$ | 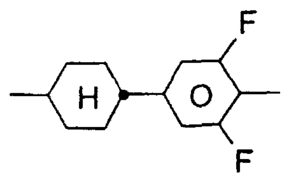 | H | F |
| $C_2H_5CH=CH$ | 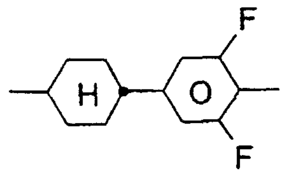 | F | F |
| $C_3H_7CH=CH$ | 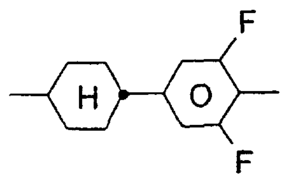 | H | H |
| $C_3H_7CH=CH$ | 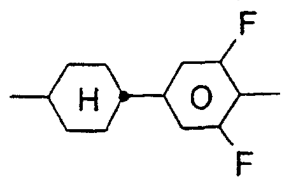 | H | F |
| $C_3H_7CH=CH$ | 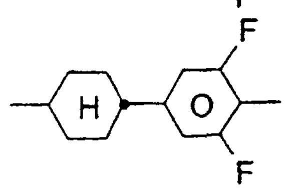 | F | F |
| $CH_2=CHCH_2CH_2$ | 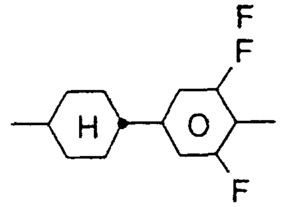 | H | H |

| R | $-(A^1-Z^1)_m-A^2-Z^2-$ | $L^1$ | $L^2$ |
|---|---|---|---|
| $CH_2=CHCH_2CH_2$ | 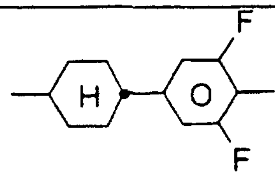 | H | F |
| $CH_2=CHCH_2CH_2$ | 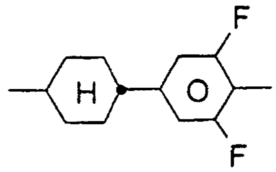 | F | F |
| $CH_3CH=CHCH_2CH_2$ | 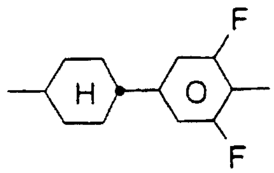 | H | H |
| $CH_3CH=CHCH_2CH_2$ | 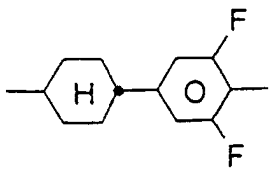 | H | F |
| $CH_3CH=CHCH_2CH_2$ | 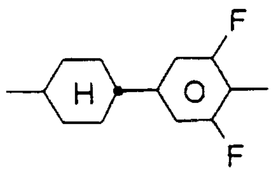 | F | F |
| $C_2H_5CH=CHCH_2CH_2$ | 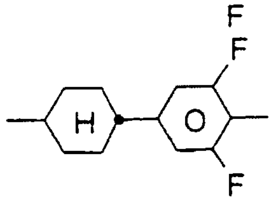 | H | H |
| $C_2H_5CH=CHCH_2CH_2$ | 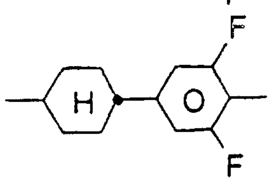 | H | F |
| $C_2H_5CH=CHCH_2CH_2$ | 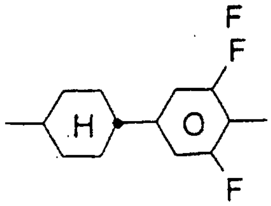 | F | F |
| $C_3H_7CH=CHCH_2CH_2$ | 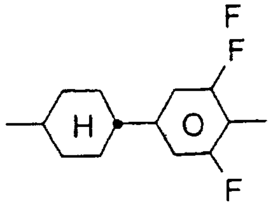 | H | H |

| R | $-(A^1-Z^1)_m-A^2-Z^2-$ | $L^1$ | $L^2$ |
|---|---|---|---|
| $C_3H_7CH=CHCH_2CH_2$ | 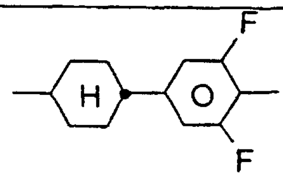 | H | F |
| $C_3H_7CH=CHCH_2CH_2$ | 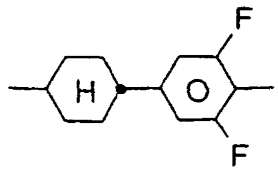 | F | F |
| $CH_2=CH$ | 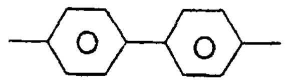 | H | H |
| $CH_2=CH$ | 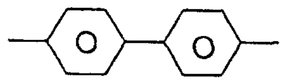 | H | F |
| $CH_2=CH$ | 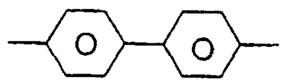 | F | F |
| $CH_3CH=CH$ | 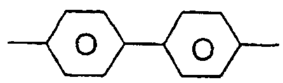 | H | H |
| $CH_3CH=CH$ | 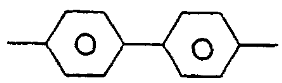 | H | F |
| $CH_3CH=CH$ | 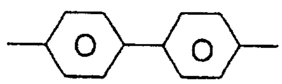 | F | F |
| $C_2H_5CH=CH$ | 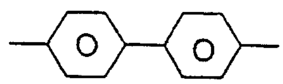 | H | H |
| $C_2H_5CH=CH$ | 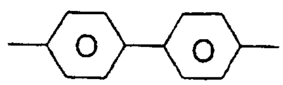 | H | F |
| $C_2H_5CH=CH$ | 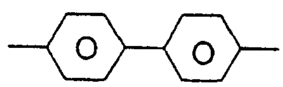 | F | F |
| $C_3H_7CH=CH$ | 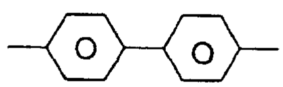 | H | H |
| $C_3H_7CH=CH$ | 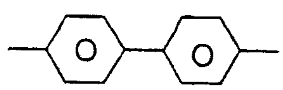 | H | F |
| $C_3H_7CH=CH$ | 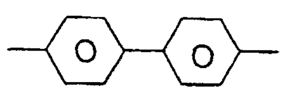 | F | F |
| $CH_2=CHCH_2CH_2$ | 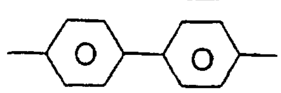 | H | H |
| $CH_2=CHCH_2CH_2$ | 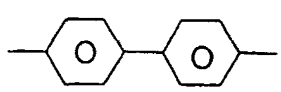 | H | F |

| R | $-(A^1-Z^1)_m-A^2-Z^2-$ | $L^1$ | $L^2$ |
|---|---|---|---|
| $CH_2=CHCH_2CH_2$ | 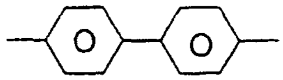 | F | F |
| $CH_3CH=CHCH_2CH_2$ | 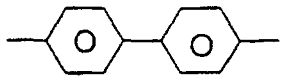 | H | H |
| $CH_3CH=CHCH_2CH_2$ | 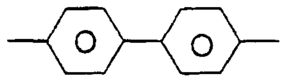 | H | F |
| $CH_3CH=CHCH_2CH_2$ | 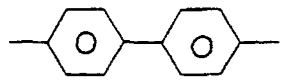 | F | F |
| $C_2H_5CH=CHCH_2CH_2$ | 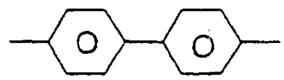 | H | H |
| $C_2H_5CH=CHCH_2CH_2$ | 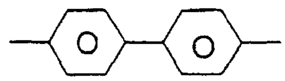 | H | F |
| $C_2H_5CH=CHCH_2CH_2$ | 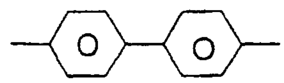 | F | F |
| $C_3H_7CH=CHCH_2CH_2$ | 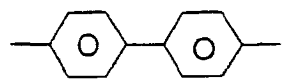 | H | H |
| $C_3H_7CH=CHCH_2CH_2$ | 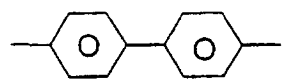 | H | F |
| $C_3H_7CH=CHCH_2CH_2$ | 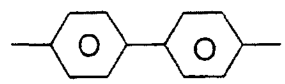 | F | F |
| $CH_2=CH$ | 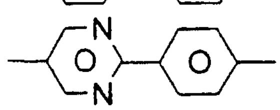 | H | H |
| $CH_2=CH$ | 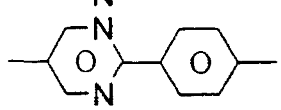 | H | F |
| $CH_2=CH$ | 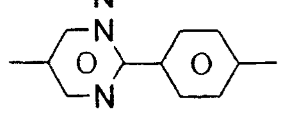 | F | F |
| $CH_3CH=CH$ | 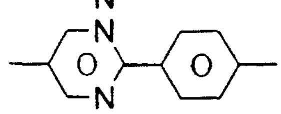 | H | H |
| $CH_3CH=CH$ | 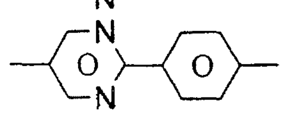 | H | F |
| $CH_3CH=CH$ | 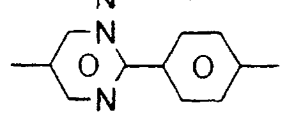 | F | F |

| R | $-(A^1-Z^1)_m-A^2-Z^2-$ | $L^1$ | $L^2$ |
|---|---|---|---|
| $CH_2=CHCH_2CH_2$ | 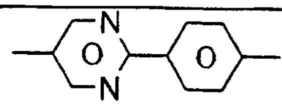 | H | H |
| $CH_2=CHCH_2CH_2$ | 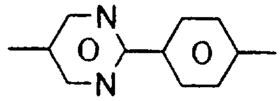 | H | F |
| $CH_2=CHCH_2CH_2$ | 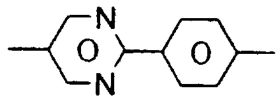 | F | F |
| $CH_3CH=CHCH_2CH_2$ | 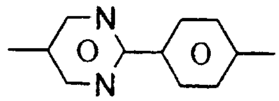 | H | H |
| $CH_3CH=CHCH_2CH_2$ | 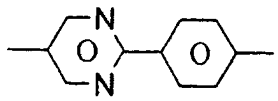 | H | F |
| $CH_3CH=CHCH_2CH_2$ | 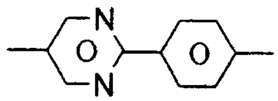 | F | F |
| $CH_2=CH$ | 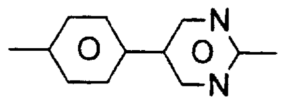 | H | H |
| $CH_2=CH$ | 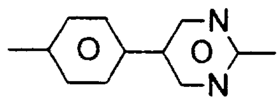 | H | F |
| $CH_2=CH$ | 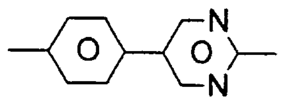 | F | F |
| $CH_3CH=CH$ | 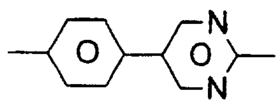 | H | H |
| $CH_3CH=CH$ | 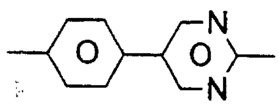 | H | F |
| $CH_3CH=CH$ | 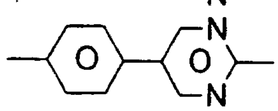 | F | F |
| $CH_2=CHCH_2CH_2$ | 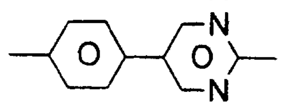 | H | H |
| $CH_2=CHCH_2CH_2$ | 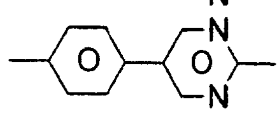 | H | F |
| $CH_2=CHCH_2CH_2$ | 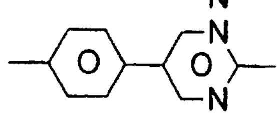 | F | F |
| $CH_3CH=CHCH_2CH_2$ | 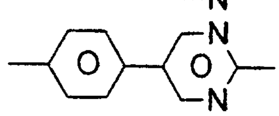 | H | H |

| R | $-(A^1-Z^1)_m-A^2-Z^2-$ | $L^1$ | $L^2$ |
|---|---|---|---|
| $CH_3CH=CHCH_2CH_2$ | 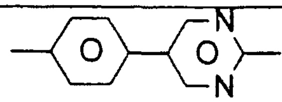 | H | F |
| $CH_3CH=CHCH_2CH_2$ | 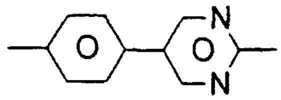 | F | F |
| $CH_2=CH$ | 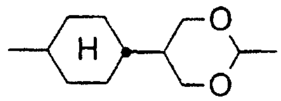 | H | H |
| $CH_2=CH$ | 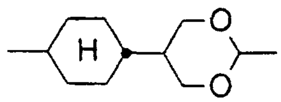 | H | F |
| $CH_2=CH$ | 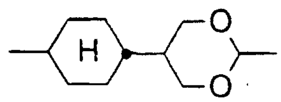 | F | F |
| $CH_3CH=CH$ | 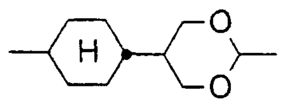 | H | H |
| $CH_3CH=CH$ | 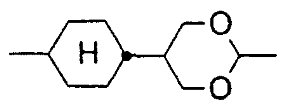 | H | F |
| $CH_3CH=CH$ | 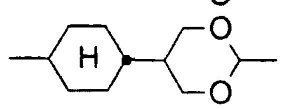 | F | F |
| $CH_2=CHCH_2CH_2$ | 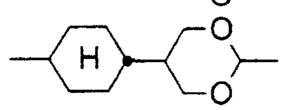 | H | H |
| $CH_2=CHCH_2CH_2$ | 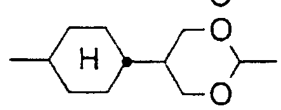 | H | F |
| $CH_2=CHCH_2CH_2$ | 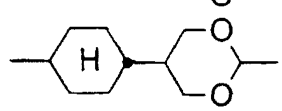 | F | F |
| $CH_3CH=CHCH_2CH_2$ | 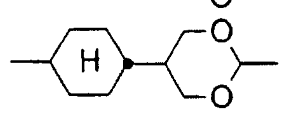 | H | H |
| $CH_3CH=CHCH_2CH_2$ | 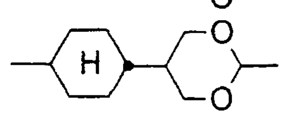 | H | F |
| $CH_3CH=CHCH_2CH_2$ | 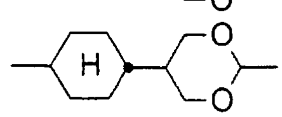 | F | F |
| $CH_2=CH$ | 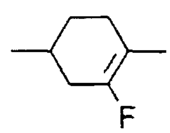 | H | H |

| R | $-(A^1-Z^1)_m-A^2-Z^2-$ | $L^1$ | $L^2$ |
|---|---|---|---|
| $CH_2=CH$ | 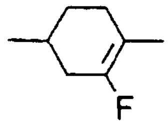 | H | F |
| $CH_2=CH$ | 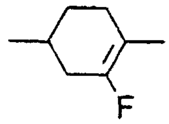 | F | F |
| $CH_3CH=CH$ | 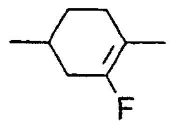 | H | H |
| $CH_3CH=CH$ | 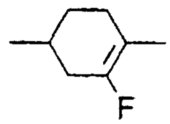 | H | F |
| $CH_3CH=CH$ | 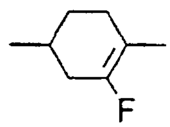 | F | F |
| $CH_2=CHCH_2CH_2$ | 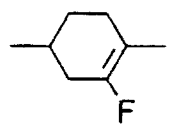 | H | H |
| $CH_2=CHCH_2CH_2$ | 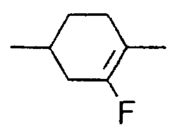 | H | F |
| $CH_2=CHCH_2CH_2$ | 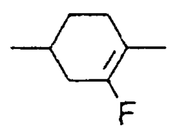 | F | F |
| $CH_3CH=CHCH_2CH_2$ | 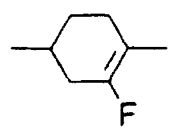 | H | H |
| $CH_3CH=CHCH_2CH_2$ | 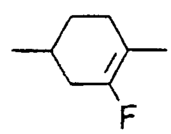 | H | F |
| $CH_3CH=CHCH_2CH_2$ | 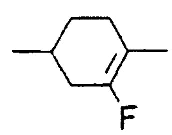 | F | F |

**Mischungsbeispiele** (Vergleichsbeispiele)

## Beispiel A

| | | | |
|---|---|---|---|
| PCH-5F | 9,0% | Klärpunkt [°C] | 87,8 |
| PCH-6F | 7,2% | Δn [589 nm, 20 °C] | 0,1074 |
| PCH-7F | 5,4% | Δε [1 kHz, 20 °C] | 8,95 |
| CCP-2OCF$_3$ | 7,2% | $\nu_{20}$ [mm$^2$·s$^{-1}$] | 15,0 |
| CCP-3OCF$_3$ | 10,8% | | |
| CCP-4OCF$_3$ | 6,3% | | |
| CCP-5OCF$_3$ | 9,9% | | |
| BCH-3F.F | 10,8% | | |
| BCH-5F.F | 9,0% | | |
| ECCP-3OCF$_3$ | 4,5% | | |
| ECCP-5OCF$_3$ | 4,5% | | |
| CBC-33F | 1,8% | | |
| CBC-53F | 1,8% | | |
| CBC-55F | 1,8% | | |
| CU-3-AN | 10,0% | | |

## Beispiel B

| | | | |
|---|---|---|---|
| PCH-5F | 9,0% | Klärpunkt [°C] | 90,8 |
| PCH-6F | 7,2% | Δn [589 nm, 20 °C] | 0,1101 |
| PCH-7F | 5,4% | Δε [1 kHz, 20 °C] | 8,32 |
| CCP-2OCF$_3$ | 7,2% | $\nu_{20}$ [mm$^2$·s$^{-1}$] | 14,0 |
| CCP-3OCF$_3$ | 10,8% | | |
| CCP-4OCF$_3$ | 6,3% | | |
| CCP-5OCF$_3$ | 9,9% | | |
| BCH-3F.F | 10,8% | | |
| BCH-5F.F | 9,0% | | |
| ECCP-3OCF$_3$ | 4,5% | | |
| ECCP-5OCF$_3$ | 4,5% | | |
| CBC-33F | 1,8% | | |
| CBC-53F | 1,8% | | |
| CBC-55F | 1,8% | | |
| CG-3-AN | 10,0% | | |

## Beispiel C

| | | | |
|---|---|---|---|
| PCH-5F | 9,0% | Klärpunkt [°C] | 89,1 |
| PCH-6F | 7,2% | Δn [589 nm, 20 °C] | +0,1070 |
| PCH-7F | 5,4% | Δε [1 kHz, 20 °C] | 8,61 |
| CCP-2OCF$_3$ | 7,2% | $\nu_{20}$ [mm$^2$·s$^{-1}$] | 15 |
| CCP-3OCF$_3$ | 10,8% | | |
| CCP-4OCF$_3$ | 6,3% | | |
| CCP-5OCF$_3$ | 9,9% | | |

(fortgesetzt)

| | | | |
|---|---|---|---|
| BCH-3F.F | 10,8% | | |
| BCH-5F.F | 9,0% | | |
| ECCP-3OCF$_3$ | 4,5% | | |
| ECCP-5OCF$_3$ | 4,5% | | |
| CBC-33F | 1,8% | | |
| CBC-53F | 1,8% | | |
| CBC-55F | 1,8% | | |
| CU-5-AN | 10,0% | | |

**Beispiel D**

| | | | |
|---|---|---|---|
| PCH-5F | 9,0% | Klärpunkt [°C] | 83,2 |
| PCH-6F | 7,2% | Δn [589 nm, 20 °C] | 0,1055 |
| PCH-7F | 5,4% | Δε [1 kHz, 20 °C] | 8,80 |
| CCP-2OCF$_3$ | 7,2% | $\nu_{20}$ [mm$^2$·s$^{-1}$] | 15 |
| CCP-3OCF$_3$ | 10,8% | | |
| CCP-4OCF$_3$ | 6,3% | | |
| CCP-5OCF$_3$ | 9,9% | | |
| BCH-3F.F | 10,8% | | |
| BCH-5F.F | 9,0% | | |
| ECCP-3OCF$_3$ | 4,5% | | |
| ECCP-5OCF$_3$ | 4,5% | | |
| CBC-33F | 1,8% | | |
| CBC-53F | 1,8% | | |
| CBC-55F | 1,8% | | |
| CU-2-AN | 10,0% | | |

**Beispiel E**

| | | | |
|---|---|---|---|
| PCH-5F | 9,0% | Klärpunkt [°C] | 86,8 |
| PCH-6F | 7,2% | Δn [589 nm, 20 °C] | 0,1206 |
| PCH-7F | 5,4% | Δε [1 kHz, 20 °C] | 9,88 |
| CCP-2OCF$_3$ | 7,2% | $\nu_{20}$ [mm$^2$·s$^{-1}$] | 14 |
| CCP-3OCF$_3$ | 10,8% | | |
| CCP-4OCF$_3$ | 6,3% | | |
| CCP-5OCF$_3$ | 9,9% | | |
| BCH-3F.F | 10,8% | | |
| BCH-5F.F | 9,0% | | |
| ECCP-3OCF$_3$ | 4,5% | | |
| ECCP-5OCF$_3$ | 4,5% | | |
| CBC-33F | 1,8% | | |
| CBC-53F | 1,8% | | |
| CBC-55F | 1,8% | | |
| PU-3-AN | 10,0% | | |

**Beispiel F**

| | | | |
|---|---|---|---|
| PCH-5F | 9,0 % | Klärpunkt [°C] | 85,2 |
| PCH-6F | 7,2 % | Δn [589 nm, 20 °C] | 0,1051 |
| PCH-7F | 5,4 % | Δε [1 kHz, 20 °C] | 10,71 |
| CCP-2OCF$_3$ | 7,2 % | $\nu_{20}$ [$mm^2 \cdot s^{-1}$] | 15 |
| CCP-3OCF$_3$ | 10,8 % | | |
| CCP-4OCF$_3$ | 6,3 % | | |
| CCP-5OCF$_3$ | 9,9 % | | |
| BCH-3F.F | 10,8 % | | |
| BCH-5F.F | 9.0 % | | |
| ECCP-3OCF$_3$ | 4,5 % | | |
| ECCP-5OCF$_3$ | 4,5 % | | |
| CBC-33F | 1,8 % | | |
| CBC-53F | 1,8 % | | |
| CBC-55F | 1,8 % | | |
| DU-3-AN | 10,0 % | | |

**Beispiel G**

| | | | |
|---|---|---|---|
| PCH-6F | 7,2 % | Klärpunkt [°C] | +93,8 |
| PCH-7F | 5,4 % | Δn [589,3 nm, 20 °C] | +0,1146 |
| CCP-20CF$_3$ | 7,2 % | Δε [1kHz, 20 °C] | +7,6 |
| CCP-30CF$_3$ | 10,8 % | $\nu_{20}$ [$mm^2 \cdot s^{-1}$] | 14 |
| CCP-40CF$_3$ | 6,3 % | | |
| PCH-5F | 9,0 % | | |
| CCP-50CF$_3$ | 9,9 % | | |
| BCH-3F.F | 10,8 % | | |
| BCH-5F.F | 9,0 % | | |
| ECCP-30CF$_3$ | 4,5 % | | |
| ECCP-50CF$_3$ | 4,5 % | | |
| CBC-33F | 1,8 % | | |
| CBC-53F | 1,8 % | | |
| CBC-55F | 1,8 % | | |
| CP-V-AN | 10,0 % | | |

**Beispiel H**

| | | | |
|---|---|---|---|
| BCH-3F.F | 10,8 % | Klärpunkt [°C] | 98,5 |
| BCH-5F.F | 9,0 % | Δn [589,3 nm, 20 °C] | +0,1162 |
| ECCP-30CF$_3$ | 4,5 % | Δε [1kHz, 20 °C] | +8,2 |
| ECCP-50CF$_3$ | 4,5 % | $\nu_{20}$ [$mm^2 \cdot s^{-1}$] | 14 |
| CBC-33F | 1,8 % | | |
| CBC-53F | 1,8 % | | |
| CBC-55F | 1,8 % | | |
| PCH-6F | 7,2 % | | |
| PCH-7F | 5,4 % | | |

(fortgesetzt)

| | | | |
|---|---|---|---|
| CCP-20CF$_3$ | 7,20 % | | |
| CCP-30CF$_3$ | 10,80 % | | |
| CCP-40CF$_3$ | 6,30 % | | |
| CCP-50CF$_3$ | 9,90 % | | |
| PCH-5F | 9,00 % | | |
| CP-1V-AN | 10,00 % | | |

Beispiel I

| | | | |
|---|---|---|---|
| PCH-6F | 7,20 % | Klärpunkt [°C] | +95,2 |
| PCH-7F | 5,40 % | Δn [589,3 nm, 20 °C] | +0,1133 |
| CCP-20CF$_3$ | 7,20 % | Δε [1kHz, 20 °C] | +7,5 |
| CCP-30CF$_3$ | 10,80 % | $\nu_{20}$ [$mm^2 \cdot s^{-1}$] | 15 |
| CCP-40CF$_3$ | 6,30 % | | |
| PCH-5F | 9,00 % | | |
| CCP-50CF$_3$ | 9,90 % | | |
| BCH-3F.F | 10,80 % | | |
| BCH-5F.F | 9,00 % | | |
| ECCP-30CF$_3$ | 4,50 % | | |
| ECCP-50CF$_3$ | 4,50 % | | |
| CBC-33F | 1,80 % | | |
| CBC-53F | 1,80 % | | |
| CBC-55F | 1,80 % | | |
| CP-V2-AN | 10,00 % | | |

**Beispiel J**

| | | | |
|---|---|---|---|
| BCH-3F.F | 10,81 % | Klärpunkt [°C] | 86,5 |
| BCH-5F.F | 9,01 % | Δn [589,3 nm, 20 °C] | +0,1087 |
| ECCP-30CF$_3$ | 4,50 % | Δε [1kHz, 20 °C] | +8,6 |
| ECCP-50CF$_3$ | 4,50 % | | |
| CBC-33F | 1,80 % | | |
| CBC-53F | 1,80 % | | |
| CBC-55F | 1,80 % | | |
| PCH-6F | 7,21 % | | |
| PCH-7F | 5,40 % | | |
| CCP-20CF$_3$ | 7,20 % | | |
| CCP-30CF$_3$ | 10,81 % | | |
| CCP-40CF$_3$ | 6,30 % | | |
| CCP-50CF$_3$ | 9,91 % | | |
| PCH-5F | 9,01 % | | |
| CU-V-AN | 9,94 % | | |

**Beispiel K**

| | | | |
|---|---|---|---|
| PCH-6F | 7,20 | Klärpunkt [°C] | 87,8 |
| PCH-7F | 5,40 | Δn [589,3 nm, 20 °C] | +0,1077 |
| CCP-20CF$_3$ | 7,20 | Δε [1kHz, 20 °C] | +8,3 |
| CCP-30CF$_3$ | 10,80 | $\nu_{20}$ [$mm^2 \cdot s^{-1}$] | 15 |
| CCP-40CF$_3$ | 6,30 | | |
| PCH-5F | 9,00 | | |
| CCP-50CF$_3$ | 9,90 | | |
| BCH-3F.F | 10,80 | | |
| BCH-5F.F | 9,00 | | |
| ECCP-30CF$_3$ | 4,50 | | |
| ECCP-50CF$_3$ | 4,50 | | |
| CBC-33F | 1,80 | | |
| CBC-53F | 1,80 | | |
| CBC-55F | 1,80 | | |
| CU-V2-AN | 10,00 | | |

**Patentansprüche**

1. Acetylenderivate der Formel I

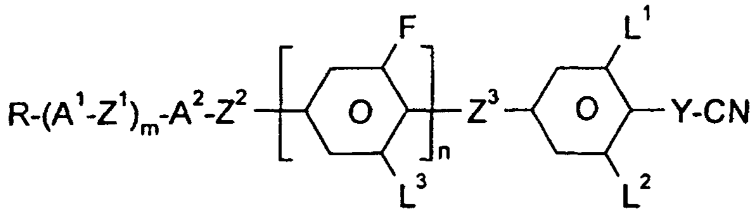

I

worin

R einen unsubstituierten, einen einfach durch CN oder $CF_3$ oder einen mindestens einfach durch Halogen substituierten Alkyl- oder Alkenylrest mit 1 bis 15 C-Atomen, wobei in diesen Resten auch eine oder mehrere $CH_2$-Gruppen jeweils unabhängig voneinander durch -O-, -S-,

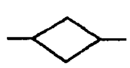,

-CO-, -CO-O-, -O-CO- oder -O-CO-O- so ersetzt sein können, daß O-Atome nicht direkt miteinander verknüpft sind,

$A^1$ und $A^2$ jeweils unabhängig voneinander

(a) trans-1,4-Cyclohexylenrest, worin auch eine oder mehrere nicht benachbarte $CH_2$-Gruppen durch -O- und/oder -S- ersetzt sein können,

(b) 1,4-Phenylenrest, worin auch eine oder zwei CH-Gruppen durch N ersetzt sein können,

(c) trans-1,4-Cyclohexenylen,

(d) Rest aus der Gruppe 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,

wobei die Reste (a) bis (c) durch Fluor oder $CH_3$ ein- oder zweifach substituiert sein können,

$Z^1$, $Z^2$ und $Z^3$ jeweils unabhängig voneinander -CO-O-, -O-CO-, $-CH_2O-$, $-OCH_2-$, $-CH_2CH_2-$, -CH=CH-, -C≡C-, $-(CH_2)_4-$, $-OCF_2-$, $-CF_2O-$, $-CH=CH-CH_2CH_2-$ oder eine Einfachbindung,

m 0, 1 oder 2,

n 0 oder 1,

$L^1$, $L^2$ und $L^3$ jeweils unabhängig voneinander H oder F,

Y -C≡C-, -CH=CH-, -CF=CH-, -CH=CF- oder -CF=CF-

bedeuten, mit der Maßgabe, daß im Fall Y = -C≡C- oder -CH=CH-

a) n = 1 und $Z^3$ eine Einfachbindung, oder

b) $L^1$ und/oder $L^2$ Fluor, oder

c) R Alkenyl oder Alkenyloxy mit 2 bis 12 C-Atomen, oder

d) $A^2$ einen Pyrimidinrest oder einen mit Fluor oder $CH_3$ ein- oder zweifach substituierten trans-1,4-Cyclohexylenrest, oder

e) einer der Reste $Z^1$, $Z^2$ oder $Z^3$ -CO-O-
bedeutet, und im Fall Y = -C≡C- zusätzlich

f) n = 1 und $L^3$ = F oder

g) mindestens einer der Reste $Z^1$, $Z^2$ und $Z^3$ $-CF_2O-$ oder $-OCF_2-$ oder

h) $A^2$ 3,5-Difluor-1,4-phenylen bedeutet.

**2.** Verbindungen der Formel I1,

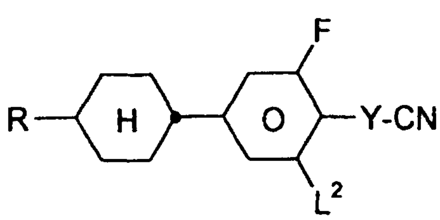

I1

worin
R, Y und L2 die in Anspruch 1 angegebenen Bedeutungen haben.

**3.** Verbindungen der Formel 12,

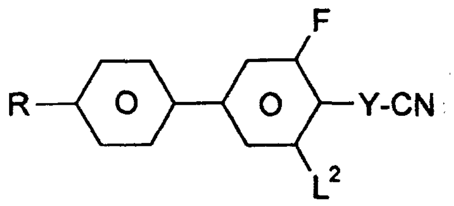

I2

worin
R, Y und $L^2$ die in Anspruch 1 angegebenen Bedeutungen haben.

**4.** Verbindungen der Formel I3,

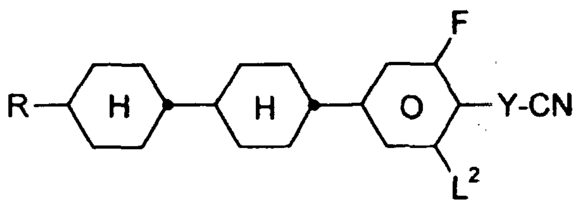

I3

worin
R, Y und $L^2$ die in Anspruch 1 angegebenen Bedeutungen haben.

**5.** Verbindungen der Formel

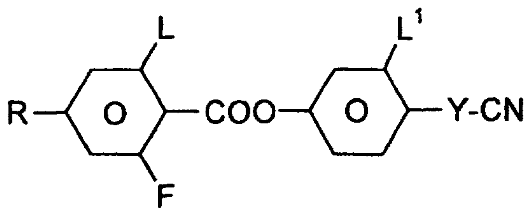

worin
L H oder F
R, Y und $L^1$ die in Anspruch 1 angegebenen Bedeutungen haben.

**6.** Verbindungen der Formel I16,

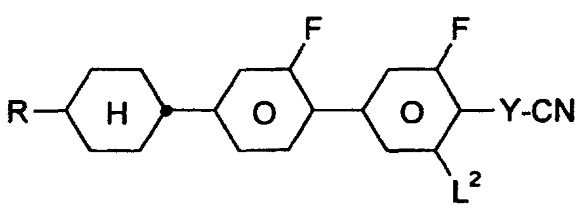

I16

worin
R, Y und $L^2$ die in Anspruch 1 angegebenen Bedeutungen haben.

**7.** Verbindungen nach einem der Ansprüche 1 bis 6, **dadurch gekennzeichnet, daß** Y -C≡C- oder -CF=CF ist.

**8.** Verbindungen nach einem der Ansprüche 1 bis 7, **dadurch gekennzeichnet, daß** R Alkenyl oder Alkenyloxy mit

2 bis 12 C-Atomen bedeutet.

**9.** Verwendung von Verbindungen der Formel I nach Anspruch 1 als Komponenten flüssigkristalliner Medien.

**10.** Flüssigkristallines Medium mit mindestens zwei flüssigkristallinen Komponenten, **dadurch gekennzeichnet, daß** es mindestens eine Verbindung der Formel I enthält.

**11.** Flüssigkristall-Anzeigeelement, **dadurch gekennzeichnet, daß** es ein flüssigkristallines Medium nach Anspruch 10 enthält.

**12.** Elektrooptisches Anzeigeelement, **dadurch gekennzeichnet, daß** es als Dielektrikum ein flüssigkristallines Medium nach Anspruch 10 enthält.

## Claims

**1.** Acetylene derivatives of the formula I

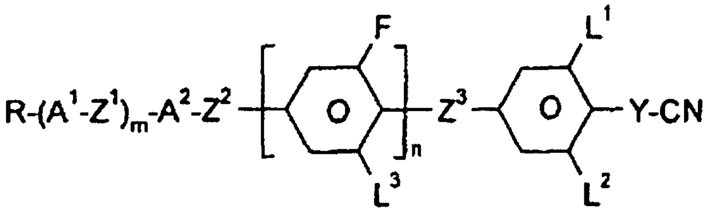

I

in which

R denotes an alkyl or alkenyl radical having 1 to 15 C atoms which is unsubstituted, monosubstituted by CN or $CF_3$ or at least monosubstituted by halogen, where, in addition, one or more $CH_2$ groups in these radicals may each be replaced, independently of one another, by -O-, -S-,

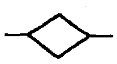

,

-CO-, -CO-O-, -O-CO- or -O-CO-O- in such a way that O atoms are not linked directly to one another,

$A^1$ and $A^2$ each, independently of one another, denote

(a) trans-1,4-cyclohexylene radical, in which, in addition, one or more non-adjacent $CH_2$ groups may be replaced by -O- and/or -S-,

(b) 1,4-phenylene radical, in which, in addition, one or two CH groups may be replaced by N,

(c) trans-1,4-cylohexenylene,

(d) radical from the group 1,4-bicyclo(2,2,2)octylene, piperidine-1,4-diyl, naphtalene-2,6-diyl, decahydronaphthalene-2,6-diyl and 1,2,3,4-tetrahydronaphthalene-2,6-diyl,

where the radicals (a) to (c) may be mono- or disubstituted by fluorine or $CH_3$,

$Z^1$, $Z^2$ and $Z^3$ each, independently of one another, denote -CO-O-, -O-CO-, $-CH_2O-$, $-OCH_2-$, $-CH_2CH_2-$, -CH=CH-, -C≡C-, $-(CH_2)_4-$, $-OCF_2-$, $-CF_2O-$, $-CH=CH-CH_2CH_2-$ or a single bond,

m denotes 0, 1 or 2,

n denotes 0 or 1,

$L^1$, $L^2$ and $L^3$ each, independently of one another, denote H or F,

Y denotes -C≡C-, -CH=CH-, -CF=CH-, -CH=CF- or -CF=CF-,

with the proviso that, in the case where Y = -C≡C- or -CH=CH-,

a) n = 1 and $Z^3$ denotes a single bond, or

b) $L^1$ and/or $L^2$ denote fluorine, or

c) R denotes alkenyl or alkenyloxy having 2 to 12 C atoms, or

d) $A^2$ denotes a pyrimidine radical or a trans-1,4-cyclohexylene radical which is mono- or disubstituted by fluorine or $CH_3$, or

e) one of the radicals $Z^1$, $Z^2$ or $Z^3$ denotes -CO-O-,
and, in the case where Y = -C≡C-, additionally

f) n = 1 and $L^3$ = F or

g) at least one of the radicals $Z^1$, $Z^2$ and $Z^3$ denotes $-CF_2O-$ or $-OCF_2-$ or

h) $A^2$ denotes 3,5-difluoro-1,4-phenylene.

**2.** Compounds of the formula I1

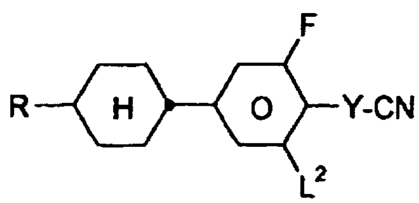

I1

in which
R, Y and $L^2$ have the meanings indicated in Claim 1.

**3.** Compounds of the formula I2

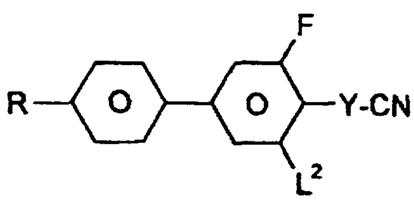

I2

in which
R, Y and $L^2$ have the meanings indicated in Claim 1.

**4.** Compounds of the formula I3

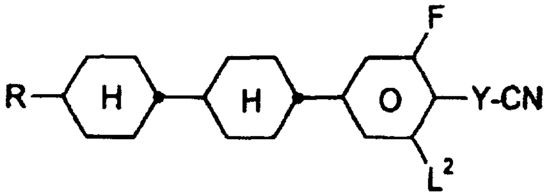

I3

in which
R, Y and $L^2$ have the meanings indicated in Claim 1.

**5.** Compounds of the formula

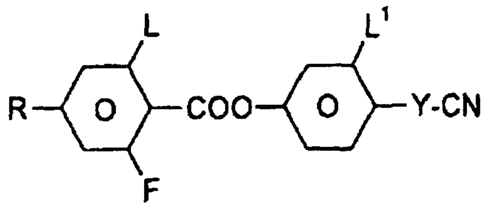

in which

L denotes H or F

R, Y and $L^1$ have the meanings indicated in Claim 1.

**6.** Compounds of the formula I16

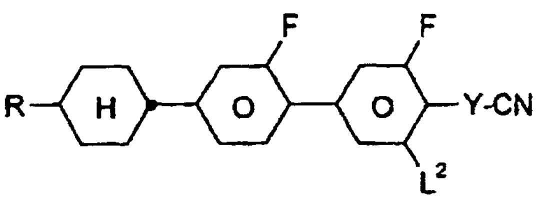

I16

in which
R, Y and $L^2$ have the meanings indicated in Claim 1.

**7.** Compounds according to one of Claims 1 to 6, **characterised in that** Y is -C≡C- or -CF=CF-.

**8.** Compounds according to one of Claims 1 to 7, **characterised in that** R denotes alkenyl or alkenyloxy having 2 to 12 C atoms.

**9.** Use of compounds of the formula I according to Claim 1 as components of liquid-crystalline media.

**10.** Liquid-crystalline medium having at least two liquid-crystalline components, **characterised in that** it comprises at least one compound of the formula I.

**11.** Liquid-crystal display element, **characterised in that** it contains a liquid-crystalline medium according to Claim 10.

**12.** Electro-optical display element, **characterised in that** it contains, as dielectric, a liquid-crystalline medium according to Claim 10.

## Revendications

**1.** Dérivés d'acétylène de la formule I

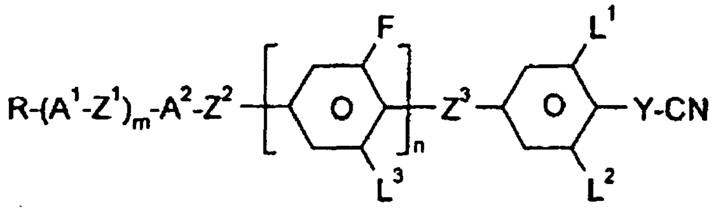

I

dans laquelle

R représente un radical alkyle ou alkényle comportant de 1 à 15 atomes de C, lequel radical est non substitué, monosubstitué par CN ou $CF_3$ ou au moins monosubstitué par halogène, où, en plus, un ou plusieurs groupes $CH_2$ dans ces radicaux peuvent chacun être remplacés, indépendamment les uns des autres, par -O-, -S-,

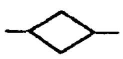,

-CO-, -CO-O-, -O-CO- ou -O-CO-O- de telle sorte que des atomes de O ne soient pas liés directement les uns aux autres,

$A^1$ et $A^2$ représentent, chacun indépendamment l'un de l'autre,

(a) un radical trans-1,4-cyclohexylène, où, en plus, un ou plusieurs groupes $CH_2$ non adjacents peuvent être remplacés par -O- et/ou -S-,

(b) un radical 1,4-phénylène, où, en plus, un ou deux groupes CH peuvent être remplacés par N,

(c) trans-1,4-cyclohexénylène,

(d) un radical pris parmi le groupe 1,4-bicyclo(2,2,2)octylène, pipéridine-1,4-diyle, naphthalène-2,6-diyle, décahydronaphthalène-2,6-diyle et 1,2,3,4-tétrahydronaphthalène-2,6-diyle,

où les radicaux (a) à (c) peuvent être mono- ou disubstitués par fluor ou $CH_3$,

$Z^1$, $Z^2$ et $Z^3$ représentent, chacun indépendamment les uns des autres, -CO-O-, -O-CO-, $-CH_2O-$, $-OCH_2-$, $-CH_2CH_2-$, -CH=CH-, -C≡C-, $-(CH_2)_4-$, $-OCF_2-$, $-CF_2O-$, $-CH=CH-CH_2CH_2-$ ou une liaison simple,

m représente 0, 1 ou 2,

n représente 0 ou 1,

$L^1$, $L^2$ et $L^3$ représentent, chacun indépendamment les uns des autres, H ou F,

Y représente -C≡C-, -CH=CH-, -CF=CH-, -CH=CF- ou -CF=CF-,

étant entendu que, dans le cas où Y = -C≡C- ou -CH=CH-,

a) n = 1 et $Z^3$ représente une liaison simple, ou

b) $L^1$ et/ou $L^2$ représentent fluor, ou

c) R représente alkényle ou alkényloxy comportant de 2 à 12 atomes de C, ou

d) $A^2$ représente un radical pyrimidine ou un radical trans-1,4-cyclohexylène, lequel radical est mono- ou disubstitué par fluor ou $CH_3$, ou

e) l'un des radicaux $Z^1$, $Z^2$ ou $Z^3$ représente -CO-O-,
et, dans le cas où Y = -C≡C-, de façon additionnelle

f) n = 1 et $L^3$ = F ou

g) au moins l'un des radicaux $Z^1$, $Z^2$ et $Z^3$ représente $-CF_2O-$ ou $-OCF_2-$ ou

h) $A^2$ représente 3,5-difluoro-1,4-phénylène.

**2.** Composés de la formule I1

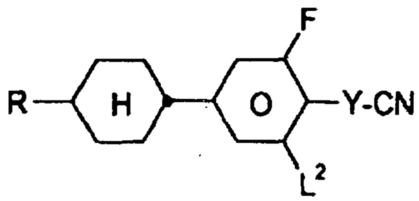

I1

dans laquelle
R, Y et $L^2$ présentent les significations qui sont indiquées dans la revendication 1.

**3.** Composés de la formule I2

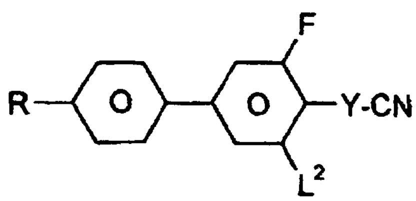

I2

dans laquelle
R, Y et $L^2$ présentent les significations qui sont indiquées dans la revendication 1.

**4.** Composés de la formule I3

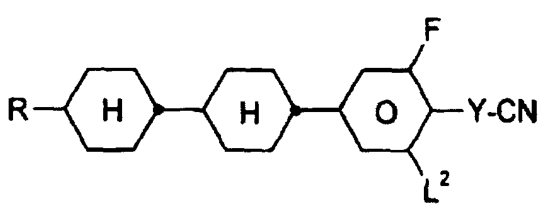

I3

dans laquelle
R, Y et $L^2$ présentent les significations qui sont indiquées dans la revendication 1.

**5.** Composés de la formule

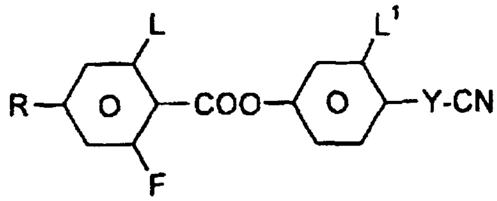

dans laquelle

L représente H ou F

R, Y et $L^1$ présentent les significations qui sont indiquées dans la revendication 1.

**6.** Composés de la formule I16

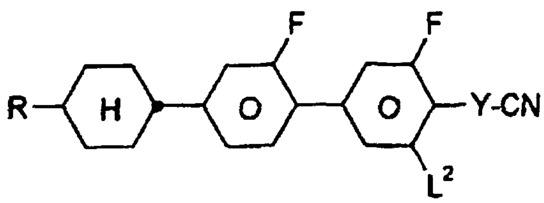

I16

dans laquelle

R, Y et $L^2$ présentent les significations qui sont indiquées dans la revendication 1.

**7.** Composés selon l'une des revendications 1 à 6, **caractérisés en ce que** Y est -C≡C- ou -CF=CF-.

**8.** Composés selon l'une des revendications 1 à 7, **caractérisés en ce que** R représente alkényle ou alkényloxy comportant de 2 à 12 atomes de C.

**9.** Utilisation de composés de la formule I selon la revendication 1 en tant que composants de milieux de cristaux liquides.

**10.** Milieu de cristaux liquides comportant au moins deux composants de cristaux liquides, **caractérisé en ce qu'**il comprend au moins un composé de la formule I.

**11.** Elément d'affichage à cristaux liquides, **caractérisé en ce qu'**il contient un milieu de cristaux liquides selon la revendication 10.

**12.** Elément d'affichage électro-optique, **caractérisé en ce qu'**il contient, en tant que diélectrique, un milieu de cristaux liquides selon la revendication 10.